# EUROPEAN PATENT SPECIFICATION

(11) **EP 2 755 545 B1**
(45) Date of publication and mention of the grant of the patent: **31.10.2018**
(21) Application number: 12832554.5
(22) Date of filing: 13.09.2012
(51) Int. Cl.: A61B 1/012, A61B 1/008, A61B 90/00, A61M 25/01

(54) **OPERATIVE ELEMENT SUPPORT STRUCTURE**
TRÄGERSTRUKTUR FÜR EIN BETRIEBSELEMENT
STRUCTURE DE SUPPORT D'ÉLÉMENT ACTIF

(30) Priority: 13.09.2011 US 201161534318 P
(43) Date of publication of application: 23.07.2014
(60) Divisional application: 14190057.1
(73) Proprietor: Covidien LP, Mansfield, MA 02048 (US)
(72) Inventor: CHANG, Scott F., Sunnyvale, California 94085 (US); HUSZAR, Hillary K., Sunnyvale, California 94085 (US); EBRAHIMIAN, Mehdi, Sunnyvale, California 94085 (US); SULLIVAN, Randall D., Sunnyvale, California 94085 (US); MATA, JR. Gilbert, Sunnyvale, California 94085 (US); GWERDER, Eric J., Sunnyvale, California 94085 (US); UTLEY, David S., Sunnyvale, California 94085 (US)
(74) Representative: Soames, Candida Jane
(86) International application number: PCT/US2012/055106
(87) International publication number: WO 2013/040175

(56) References cited:
- JP-A- 2006 141 725
- JP-A- 2009 261 642
- US-A1- 2003 176 766
- US-A1- 2005 234 297
- US-A1- 2008 281 299
- US-A1- 2009 125 002
- US-A1- 2009 198 251
- US-A1- 2009 229 615
- US-A1- 2010 125 164
- US-A1- 2011 099 773

## Description

### BACKGROUND

A variety of techniques exist for diagnosing and providing therapy to target treatment areas in the body, such as tissue. One traditional method for diagnosing and/or providing therapy to a target treatment area involves the use of an endoscope or the like to move operative elements, such as ablation devices, through a body lumen and to a target treatment area. In some embodiments, the operative element is attached to the distal end of the endoscope, and the endoscope is used to guide the operative element through the body lumen and to the target treatment area. An example of a device for such releasable mount devices is disclosed in US 2005/0234297 A1.

A problem exists, however, in that it may be difficult to attach the operative element to the distal end of an endoscope using some of the previously known attachment mechanisms. Also, some attachment mechanisms may risk damaging the endoscope when being attached to the distal end of the endoscope. Additionally, some mechanisms for attachment require relatively permanent attachment of the operative element to the endoscope, which may limit future use of both the endoscope and the operative element. Further, some manners of attaching a operative element to an endoscope do not adequately maintain the operative element in a stationary position on the endoscope. For example, the operative element may undesirably slide along the axial length of the endoscope or rotate about the endoscope as the operative element is moved through the body lumen.

There may thus be a need for apparatus that may overcome the above and/or other disadvantages of known systems and methods.

### SUMMARY

The disadvantages are overcome by an apparatus as defined in claim 1. The dependent claims define further preferred embodiments. Methods, systems, and devices are described for providing treatment to a target site, such as a tissue site within a body lumen, utilizing a releasable mount device configured for coupling an operative element, such as an ablation element, with a therapeutic of diagnostic device, such as an endoscope. The releasable mount device may include a substantially tubular base having a slot extending longitudinally from the distal end of the tubular base to the distal end of the tubular base. The tubular base may be coupled with the distal end of a therapeutic or diagnostic device, such as an endoscope, by passing the distal end through the slot. The width of the slot may expand to allow the distal end of the therapeutic or diagnostic device to pass through the slot and become mounted with the tubular base. A support member may be coupled with the tubular base, such as at location opposite the slot. The support member may include a ridge portion that is configured to receive a recess in an operative element to thereby couple the operative element to the support member. In some embodiments, the support member includes a planar surface configured for coupling an operative element to the support member. The tubular base may have a scalloped shape.

Some embodiments include releasable mount devices that include a tubular base that does not include a slot. The tubular base may be coupled with the distal end of a therapeutic or diagnostic device by passing the distal end through the tubular base in an axial direction. One or more gripping members may be positioned on an interior surface of the tubular base. The one or more gripping members may extend radially inwardly from the interior surface and may be configured for contacting and gripping the distal end of the therapeutic or diagnostic device. In some embodiments, the gripping members are compressible springs. A support member may be coupled with the tubular base. The support member may include a ridge portion that is configured to receive a recess in an operative element to thereby couple the operative element to the support member. In some embodiments, the support member includes a planar surface configured for coupling an operative element to the support member.

Some embodiments include releasable mount devices configured for coupling an operative element with a therapeutic or diagnostic device. The releasable mount device may include at least: a substantially tubular base configured to couple with a distal end of the therapeutic or diagnostic device comprising a slot extending in a longitudinal direction between a distal end and a proximal end of the tubular base; or a support member coupled with the tubular base and configured for coupling the operative element with the tubular base.

The tubular base may be configured to radially expand whereby a slot width of the slot may increase from a first width to a second width, the first width being less than a diameter of the distal end of the therapeutic or diagnostic device and the second width being at least equal to the diameter of the distal end of the therapeutic or diagnostic device. The tubular base may be configured to couple with the distal end of the therapeutic or diagnostic device is configured to clamp onto the distal end of the therapeutic or diagnostic device. At least one longitudinal edge of the tubular base may define the slot has a rounded surface. The releasable mount device may include operative element coupled with the support member.

The support member may include a foot portion located at the proximal end of the tubular base and a ridge portion extending from the foot portion to the distal end of the tubular base. The operative element may include a recess configured to receive the ridge portion of the support member. The support member may be coupled with the tubular base at a location opposite the slot. The at least one longitudinal edge of the tubular base may be shorter than the length of the tubular base proximate the support member. A thickness of a wall of the tubular base proximate the slot may be greater than a thickness of the wall of the tubular base proximate the support member.

The at least one longitudinal edge may include a contact member located along their entire length of the at least one longitudinal edge. The contact member may include a groove on an interior surface of the contact member. The contact member may extend away from an interior of the tubular base to form a radiused contact surface.

The support member may pivotably support the operative element. The tubular base and the support member may be monolithically formed. The support member may include a planar surface. The planar surface may extend from a distal end to the proximal end of the tubular base. The operative element may include a recess configured to receive the planar surface of the support member.

The releasable mount device may include at least one friction member positioned on an interior surface of the tubular base, the at least one friction member extending longitudinally from a distal end to a proximal end of the tubular base. The releasable mount device may include a protective guard disposed over the distal end and the proximal end of the tubular base. The releasable mount device may include an exterior coating disposed on the tubular base and the support member. Expansion of the tubular base may be entirely within an elastic range. At least one longitudinal edge of the tubular basis defining the slot may be straight and extends substantially parallel to a longitudinal axis of the tubular base.

The operative element may include an ablation device. The friction member may be compressible and configured to apply a restoring force under compression. The friction member may be a member selected from the group consisting of a compressible foam, a low durometer pad, a tacky adhesive, and a combination of the same. The friction member may cover essentially all of the inner surface of the tubular base.

The releasable mount device may include a spring fastener extending across the slot. The spring fastener may include at least a planar spring, an elastomer, or a textile.

The first width of the slot may be selected from the group consisting of about 2mm , about 3 mm, about 4 mm, about 5 mm, about 6 mm, about 7 mm, about 8 mm, about 9 mm, about 10 mm, about 11 mm, about 12 mm, about 13 mm, about 14 mm, about 15 mm, about 16 mm, about 17 mm, about 18 mm, about 19 mm, about 20 mm, about 21 mm, about 22 mm, about 23 mm, about 24 mm, and about 25 mm. The second width of the slot may be in the range of between about 8.6mm to about 13.8mm. The slot width of the slot of the tubular base may decrease from the second width to a third width, the third width being less than the diameter of the therapeutic or diagnostic device to clamp the tubular base onto the therapeutic or diagnostic device.

The tubular base may be formed of a thermoplastic or thermoset polymer. In some embodiments, the tubular base may be formed from a non-elastomeric material, such as a non-silicone material or a non-rubber material.

A portion of a wall of the tubular base adjacent the slot may be thinner than a portion of the wall of the remainder of the tubular base. A thickness of the portion of the wall of the tubular base adjacent the slot may be about 0.03 inch and a thickness of the portion of the wall of the remainder of the tubular base is about 0.06 inch. A wall of the tubular base adjacent the slot may have a thickness in the range of between about 0.005 inch and about 0.1 inch.

At least one longitudinal edge of the tubular base defining the slot may include a serif shape along its entire longitudinal length. The contact member may extend back towards the interior of the tubular base at the distal end. At least one longitudinal edge of the tubular base defining the slot may be configured to deflect in response to a contact force. The at least one longitudinal edge may be hinged.

The exterior coating may include at least a gel, a polymer, or a bioactive agent. An inner diameter of the tubular base in a non-expanded state may be essentially equal to the second width. The ablation device may include multiple radiofrequency electrode arrays for delivering sufficient energy to a tissue region in an alimentary tract to ablate the tissue region.

Some embodiment include systems that may include at least: a therapeutic or diagnostic device having a distal end; a releasable mount device mounted on the distal end of the therapeutic or diagnostic device; or an operative element coupled with the support member. The releasable mount device may include at least: a substantially tubular base configured to couple with a distal end of the therapeutic or diagnostic device comprising a slot extending in a longitudinal direction between a distal end and a proximal end of the tubular base; or a support member coupled with the tubular base and configured for coupling the operative element with the tubular base.

The tubular base may be configured to radially expand whereby a slot width of the slot increases from a first width to a second width, the first width being less than a diameter of the distal end of the therapeutic or diagnostic device and the second width being at least equal to the diameter of the distal end of the therapeutic or diagnostic device. The tubular base configured to couple with the distal end of the therapeutic or diagnostic device is configured to clamp onto the distal end of the therapeutic or diagnostic device. At least one longitudinal edge of the tubular base defining the slot may have a rounded surface.

The support member may include a foot portion located at the distal end of the tubular base and a ridge portion extending from the foot portion to the proximal end of the tubular base and the operative element includes a recess configured to receive the ridge portion of the support member.

Some embodiments include methods of providing treatment to a target treatment area including at least: providing a releasable mount device; mounting the releasable mount device on the distal end of a therapeutic or diagnostic device by moving the distal end of the therapeutic or diagnostic device through the slot in a transverse direction; advancing the therapeutic or diagnostic device into a body lumen; or moving the releasable mount device toward the target treatment area. The releasable mount device may include at least: a substantially tubular base configured to couple with a distal end of the therapeutic or diagnostic device comprising a slot extending in a longitudinal direction between a distal end and a proximal end of the tubular base; or a support member coupled with the tubular base and configured for coupling an operative element with the tubular base.

The tubular base may be configured to radially expand whereby a slot width of the slot increases from a first width to a second width, the first width being less than a diameter of the distal end of the therapeutic or diagnostic device and the second width being at least equal to the diameter of the distal end of the therapeutic or diagnostic device. The tubular base configured to couple with the distal end of the therapeutic or diagnostic device may be configured to clamp onto the distal end of the therapeutic or diagnostic device. At least one longitudinal edge of the tubular base defining the slot may have a rounded surface. The releasable mount device may include the operative element coupled with the support member and the method further comprises activating the operative element.

Some embodiments include releasable mount devices configured for coupling an operative element with a therapeutic or diagnostic device. The releasable mount devices may include at least: a substantially tubular base configured to couple with a distal end of the therapeutic or diagnostic device having a proximal end, a distal end, and a central longitudinal axis, an interior diameter of the tubular base being larger than the diameter of the distal end of the therapeutic or diagnostic device; one or more gripping members positioned on an interior surface of the tubular base, the one or more gripping members extending radially inward from the interior surface and comprising a friction surface; and/or a support member coupled with the tubular base, the support member comprising a rigid support surface configured for coupling with the operative element.

The one or more gripping members may extend from the distal end of the tubular base toward the proximal end of the tubular base. The one or more gripping members may extend continuously from the distal end of the tubular base to the proximal end of the tubular base and are aligned essentially parallel to the central longitudinal axis of the tubular base.

The one or more gripping members may be a compressible spring configured to apply a retaining force to the therapeutic or diagnostic device. A proximal end of the spring may have a smooth leading edge. The one or more gripping members may have a ramped leading edge. The compressible spring may be an arcuate leaf spring. The compressible spring may extend radially inwardly by more than 0.03 inches when uncompressed. The compressible spring may be formed of a material selected from a group consisting of a metal, an elastomer, a thermoplastic, a thermoset polymer, a spring steel or shape memory alloy, and a combination of the same.

The one or more gripping members may be made of a relatively low durometer material. The relatively low durometer material may be polyether block amide.

The tubular base may be a solid body having a continuous circumference. The interior surfaces of the one or more gripping members may define an expandable diameter, a non-expanded diameter of the expandable diameter being less than the diameter of the distal end of the therapeutic or diagnostic device. The tubular base may have an interior diameter selected from the group consisting of about 4 mm, about 12 mm, about 18 mm, about 22 mm, and about 25 mm. The tubular base may include a lip at the distal end for engaging a distal end of the therapeutic or diagnostic device. At least the tubular base, one or more gripping members, or the support member may be monolithically formed.

The support member may include a planar surface. The planar surface may extend less than the length of the tubular base. The releasable mount device may include an operative element coupled with the support member. The operative element may include a recess configured to receive the planar surface of the support member. The operative element may be an ablation device. The ablation device may include multiple radiofrequency electrode arrays for delivering sufficient energy to a tissue region in an alimentary tract to ablate the tissue region. The support member may pivotably support the operative element.

Some embodiments include systems that include at least: a therapeutic or diagnostic device having a distal end; a releasable mount device mounted on the distal end of the therapeutic or diagnostic device; or an operative element coupled with the support member. The releasable mount device may include at least: a substantially tubular base configured to couple with the distal end of the therapeutic or diagnostic device having a proximal end, a distal end, and a central longitudinal axis, an interior diameter of the tubular base being larger than the diameter of the distal end of the therapeutic or diagnostic device; one or more gripping members positioned on an interior surface of the tubular base, the one or more gripping members extending radially inwardly from the interior surface and comprising a friction surface; or a support member coupled with the tubular base, the support member comprising a rigid support surface configured for coupling with an operative element. The interior surfaces of the one or more gripping members may define an expandable diameter, a non-expanded diameter of the expandable diameter being less than the diameter of the distal end of the therapeutic or diagnostic device.

Some embodiments include methods of providing treatment to a target treatment area that may include at least: providing a releasable mount device; mounting the releasable mount device to a distal end of a therapeutic or diagnostic device by passing the distal end of the therapeutic of diagnostic device completely through the distal end of the tubular base; advancing the therapeutic or diagnostic device into a body lumen; or moving the releasable mount device toward the target treatment area. The releasable mount device may include at least: a substantially tubular base having a proximal end, a distal end, and a central longitudinal axis, an interior diameter of the tubular base being larger than the diameter of the distal end of the therapeutic or diagnostic device; one or more gripping members positioned on an interior surface of the tubular base, the one or more gripping members extending radially inwardly from the interior surface and comprising a friction surface; and a support member coupled with the tubular base, the support member comprising a rigid support surface configured for coupling with an operative element. The releasable mount device may further include an operative element coupled with the support member and the method further comprises activating the operative element.

The foregoing has outlined rather broadly the features and technical advantages of examples according to the disclosure in order that the detailed description that follows may be better understood. Additional features and advantages will be described hereinafter. The conception and specific examples disclosed may be readily utilized as a basis for modifying or designing other structures for carrying out the same purposes of the present disclosure. Such equivalent constructions do not depart from the spirit and scope of the appended claims. Features which are believed to be characteristic of the concepts disclosed herein, both as to their organization and method of operation, together with associated advantages will be better understood from the following description when considered in connection with the accompanying figures. Each of the figures is provided for the purpose of illustration and description only, and not as a definition of the limits of the claims.

### BRIEF DESCRIPTION OF THE DRAWINGS

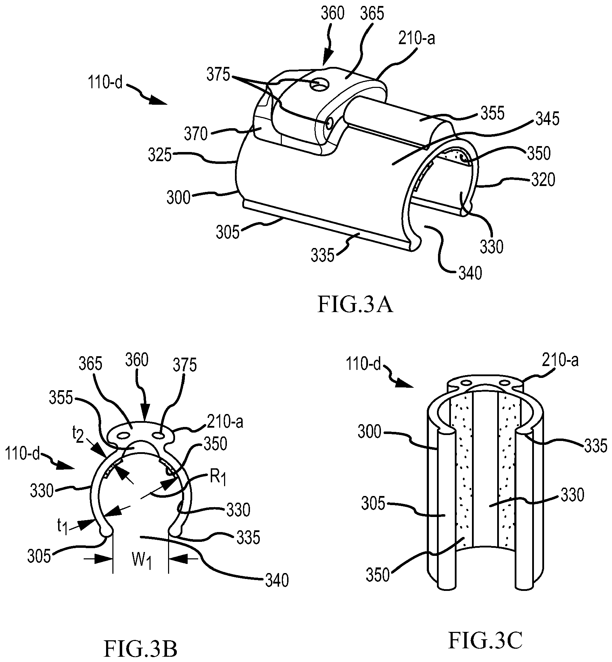

A further understanding of the nature and advantages of the embodiments may be realized by reference to the following drawings. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label. The features of the invention can best be seen in figures 3A to 4A and 14A to 15B.
FIG. 1A is a schematic diagram of a system including a therapeutic or diagnostic device and a releasable mount device configured according to various embodiments.
FIG. 1B is a schematic diagram of one specific embodiment of the system shown in FIG. 1A,
FIG. 2A is a perspective view of a distal end of a therapeutic or diagnostic device according to various embodiments.
FIG. 2B is a perspective view of a releasable mount device according to various embodiments mounted on the therapeutic or diagnostic device shown in FIG. 2A.
FIG 2C is a perspective view of a releasable mount device according to various embodiments mounted on the therapeutic or diagnostic device shown in FIG. 2A.
FIG. 2D is a perspective view of a releasable mount device coupled with an operative element according to various embodiments mounted on the therapeutic or diagnostic device shown in FIG. 2A.
FIG. 2E is a perspective view of a releasable mount device coupled with an operative element according to various embodiments mounted on the therapeutic or diagnostic device shown in FIG. 2A.
FIG. 3A is a perspective view of a releasable mount device mount according to various embodiments.
FIG. 3B is an end view of the distal end of the releasable mount device shown in FIG. 3A.
FIG. 3C is a perspective bottom view of the releasable mount device mount shown in FIG. 3A.
FIG. 4A is an enlarged cross-sectional view of a longitudinal edge of a releasable mount device having a curviplanar shape according to various embodiments.
FIG. 4B is an enlarged cross-sectional view of a longitudinal edge of a releasable mount device having a serif shape according to various embodiments.
FIG. 5A is distal end view of a releasable mount device being mounted on a therapeutic or diagnostic device according to various embodiments.
FIG. 5B is a distal end view of a releasable mount device mounted on a therapeutic or diagnostic device according to various embodiments.
FIG. 6 is a perspective view of a releasable mount device including protective guards according to various embodiments.
FIG. 7 is a perspective view of a releasable mount device including an exterior coating according to various embodiments.
FIG. 8 is a perspective view of a releasable mount device including a partial exterior coating according to various embodiments.
FIG. 9 is a perspective view of a releasable mount device according to various embodiments.
FIG. 10 is a perspective view of a releasable mount device according to various embodiments.
FIG. 11 is a perspective view of a releasable mount device according to various embodiments.
FIG. 12 is a perspective view of a releasable mount device according to various embodiments.
FIG. 13A is a perspective view of a releasable mount device according to various embodiments.
FIG. 13B is a side view of the releasable mount device shown in FIG. 13A.
FIG. 13C is distal end view of the releasable mount device shown in FIG. 13A.
FIG. 13D is a proximal end view of the releasable mount device shown in FIG. 13A. cross-sectional side view of an alternative releasable mount device with no slot and a friction member including a plurality of beads.
FIG. 14A a perspective view of a releasable mount device according to various embodiments.
FIG 14B is a cross section side view of the releasable mount device shown in FIG. 14A.
FIG. 15A is a perspective view of a releasable mount device according to various embodiments.
FIG. 15B is a cross section side view of the releasable mount device shown in FIG. 15A.
FIG. 16 is a flow diagram illustrating a method for providing treatment to a target treatment area according to various embodiments.
FIG. 17 is a flow diagram illustrating a method for providing treatment to a target treatment area according to various embodiments.

### DETAILED DESCRIPTION

Methods, systems, and devices are described for providing treatment to a target site, such as a tissue site within a body lumen, utilizing a releasable mount device configured for coupling an operative element, such as an ablation element, with a therapeutic of diagnostic device, such as an endoscope. The releasable mount device may include a substantially tubular base and a support member coupled with the tubular base. In some embodiments, the tubular base includes a slot extending longitudinally between the distal end and proximal end of the tubular base. The slot may expand in width to allow a distal end of an, for example, endoscope to be passed through the slot and thereby couple the releasable mount device with the endoscope. After the endoscope passes through the slot, the width of the slot may return to close to its original width so that the tubular base fits snuggly around the endoscope.

The support member may be coupled with the tubular base at a location opposite the slot. The support member may be provided to couple an operative element to the releasable mount device. In some embodiments, the support member includes a ridge portion and a foot portion. The foot portion may be located at a proximal end of the tubular base and the ridge portion may extend from the foot portion to the distal end of the tubular base. The support member may couple an operative element to the releasable mount device using a "keying" structure. For example, the operative element may include a recess that mates with the ridge of the support member. Other support member structures may also be used, such as a planar surface extending the length of the tubular base and which may be used to coupled an operative element to the releasable mount device.

In some embodiments, the releasable mount device includes a substantially tubular base and a support member, but the tubular base does not include a slot. The releasable mount device may be coupled with a therapeutic or diagnostic device by threading the distal end of the therapeutic or diagnostic device through the tubular base in an axial direction. The tubular base may include gripping members on the interior surface of the tubular base that extend radially inwardly. In some embodiments, the interior surface of the tubular base has a diameter larger than the diameter of the distal end of the therapeutic or diagnostic device, while the surfaces of the gripping members define a effective diameter that is smaller than the diameter of the distal end of the therapeutic or diagnostic device. When the distal end of the therapeutic or diagnostic device is inserted through the tubular base, the gripping members may be compressed radially outwardly and thereby grip the distal end and secure the releasable mount device to the therapeutic or diagnostic device. The support member may be similar or identical to the support member described above with respect to releasable mount device having a tubular base with a slot.

In various embodiments, the operative element is a device for delivering treatment at a tissue site. The operative element may be configured to delivery energy. The modes of energy may include, but are not limited to, radiofrequency, laser, microwave, ultrasound, heat, steam, and/or cryogenic. Energy delivery may be provided by appropriately configured therapeutic or diagnostic device. In some cases, the operative element may be configured to deliver a composition such as a pharmaceutical composition, biocompatible material, and/or stem cell(s). The operative element may be an implantable medical device. In various embodiments, the operative element is configured for performing an operation including, but not limited to, surgical procedures and diagnostics. The therapeutic or diagnostic device may include, but is not limited to an endoscope, a catheter, a guide wire, a trocar, a bronchoscope, or a device for implantation and/or delivery into a patient's body.

Reference may now be made in detail to exemplary embodiments, examples of which are illustrated in the accompanying drawings. While some embodiments will be described in conjunction with the exemplary embodiments, it will be understood that they are not intended to limit to those embodiments. On the contrary, the various embodiments are intended to cover alternatives, modifications and equivalents, which may be included within the spirit and scope of the various embodiments as defined by the appended claims.

For convenience in explanation and accurate definition in the appended claims, the terms "vertical" or "horizontal", "longitudinal", "transverse", "up" or "upper", "down" or "lower", and "inner" and "outer" are used to describe features of the present invention with reference to the positions of such features as displayed in the figures.

In many respects the modifications of the various figures resemble those of preceding modifications, and the same reference numerals followed by subscripts "A", "B", "C", and "D" designates corresponding parts.

The abbreviations and terminology used herein generally have their conventional meaning in the medical and electromechanical arts unless otherwise noted.

As used herein, the singular forms include plural referents, and vice versa, unless the context clearly dictates otherwise.

The term "bioactive agent" refers to an organic molecule that has activity to produce a desired effect in a biological system.

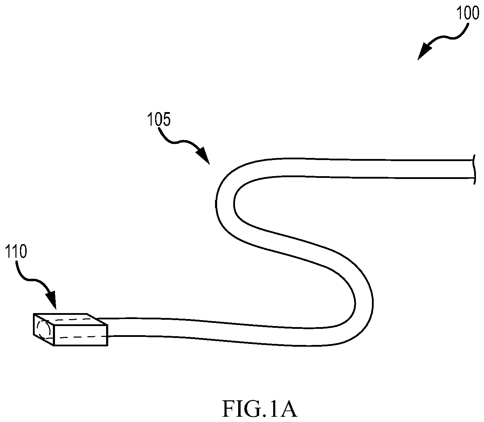

With reference to **FIG. 1A**, a schematic view of a general system 100 for delivering treatment to a target treatment is shown in accordance with various embodiments. The system 100 may include a therapeutic or diagnostic device 105 and a releasable mount device 110. The releasable mount device 110 may be releasably coupled with the therapeutic or diagnostic device 105. In some embodiments, the releasable mount 110 is releasably coupled with the distal end of the therapeutic or diagnostic device 105. As shown in FIG. 1A, the distal end of the releasable mount device 110 may be aligned with the distal end of the therapeutic or diagnostic device 105. In alternative configurations, the distal end of the releasable mount device 110 may extend beyond the distal end of the therapeutic or diagnostic device 105 or the distal end of the therapeutic or diagnostic device 105 may extend beyond the distal end of the releasable mount device 110.

The system 100 may be used for treating interior regions of the body, including, for example, tissue regions. For example, the system 100 may be configured to provide treatment to tissue located in an inner lumen of the body.

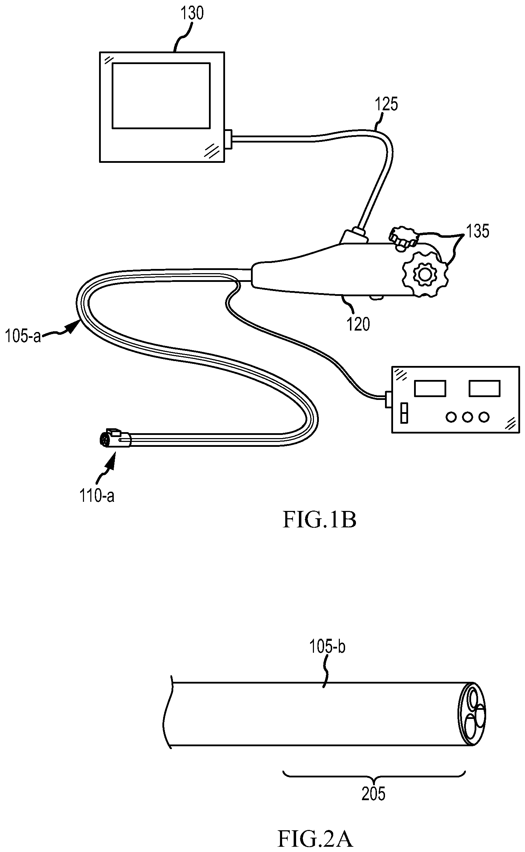

**FIG. 1B** is a schematic view of one specific embodiment of the system 100 shown in FIG. 1A. The system 100-a shown in FIG. 1B may include a releasable mount device 110-a. The releasable mount device 110-a may be supported by a therapeutic or diagnostic device 105-a. The therapeutic or diagnostic device 105-a may be, for example, an endoscope. An example of a commercially available endoscope suitable for use in various embodiments described herein is the Olympus "gastrovideoscope" model number GIF-Q160. The therapeutic or diagnostic device 105-a may include a shaft having a steerable distal end. In various embodiments, the releasable mount device 110-a is mounted on the exterior distal end of the therapeutic or diagnostic device. The therapeutic or diagnostic device may include a hub or handle 120 that includes a visual channel 125 for connecting to a video screen 130. A port (not shown) providing access to an inner working channel may be positioned within the shaft of the therapeutic or diagnostic device 105-a. Mechanisms such as dials 135 and levers (not shown) may be provided on the handle 120 to allow an operator to selectively steer the distal end of the therapeutic or diagnostic device 105-a as is well known in the arts.

As described above, the therapeutic or diagnostic device 105-a may be an endoscope. However, one may appreciate that the releasable mount device 110-a may be used with other therapeutic or diagnostic devices including but not limited to a catheter, trocar, bronchoscope, or other device.

**FIG. 2A** is an enlarged view of a distal end of a therapeutic or diagnostic device 105-b. A releasable mount device may be configured to mount over a portion 205 of the therapeutic or diagnostic device 105-b. The portion 205 may extend from an inner position on the distal end to an outer most position on the distal end of the therapeutic or diagnostic device 105-b. In various embodiments, a releasable mount device is mounted to the therapeutic or diagnostic device 105-b on the outer most position on the distal end. It may be desired to mount the releasable mount device to the outer most position on the distal end of the therapeutic or diagnostic device 105-b in order to target a specific treatment area. In various embodiments, a releasable mount device is mounted to a portion 205 that is an inner position on the distal end of the therapeutic or diagnostic device 105-b. When a releasable mount device is mounted at the inner position, there may be one or more supplemental devices positioned at the distal end of the therapeutic or diagnostic device 105-b. For example, one or more devices may include a cleaning device used in conjunction with an operative element to clean away the treated material (e.g., ablated tissue).

A releasable mount device suitable for use in various embodiments described herein may be a releasable mount device that is easy to attach and remove from an existing therapeutic or diagnostic device. In various embodiments, the therapeutic or diagnostic device is a conventional medical instrument. The releasable mount device may include a tubular base and a support member for mounting an operative element to the releasable mount device. The operative element may be any therapeutic or diagnostic device, element or structure, and may include, but is not limited to, an ablation device, a cautery device, an ultrasound therapy device, an imaging device, an ultrasound imaging probe, a transeophagael imaging probe, a cryogenic delivery device, a spray device, a probe or a therapy device, or other device suited for use with a releasable mount device.

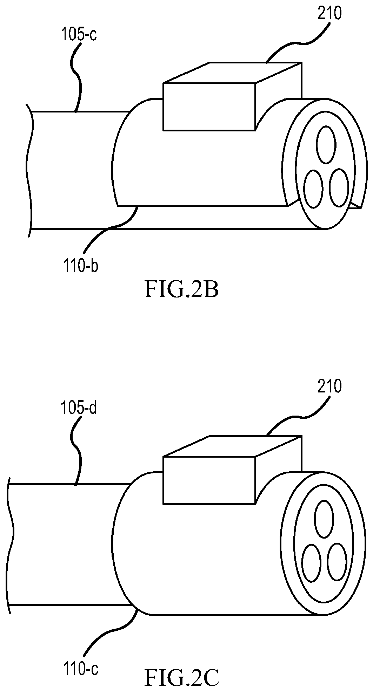

The releasable mount device may have a shape that conforms generally to the outer shape and size of the therapeutic or diagnostic device to which it may be releasably attached. For generally cylindrical therapeutic or diagnostic devices, the base may have a generally tubular shape. With reference to **FIG. 2B**, the releasable mount device 110-b may have a tubular shape with a longitudinal slot extending from a distal end to a proximal end of the releasable mount device 110-b. In such configurations, the releasable mount device 110-b may be coupled with the therapeutic or diagnostic device 105-c by passing the therapeutic or diagnostic device 105-c through the slot of the releasable mount device. With reference to **FIG. 2C**, the releasable mount device 110-c may have a tubular shape that is fully closed (i.e., does not include a slot). In such configurations, the releasable mount device 110-c may be coupled with the therapeutic or diagnostic device 105-d by passing the distal end of the therapeutic or diagnostic device 105-d through the hollow center of the releasable mount device 110-c.

With reference to both FIGs. 2B and/or 2C, the proximal and distal ends of the releasable mount device 110-b, 110-c may be generally flat and substantially parallel. The proximal and distal ends may also be angled inwardly or outwardly. It is to be appreciated that one or both ends of the releasable mount device may angle inwardly or outwardly from the top surface of the releasable mount device towards the bottom surface. Releasable mount devices 110-b, 110-c may also include a support member 210 that is configured for coupling an operative element with the releasable mount device 110-b, 110-c. The support member 210may be coupled with the base portion of the releasable mount device 110-b, 110-c and may provide a mechanism by which an operative element may be coupled with the support member 210. As shown in FIG. 2B, the support member 210 may be coupled with the releasable mount device 110-b at a location opposite the slot. FIGs. 2B and 2C show the support member 210 having a length less than the length of the base portion of the releasable mount device 110-b, 110-c, but other configurations are also possible, such as the support member 210 having a length equal to the length of the releasable mount device 110-b, 110-c.

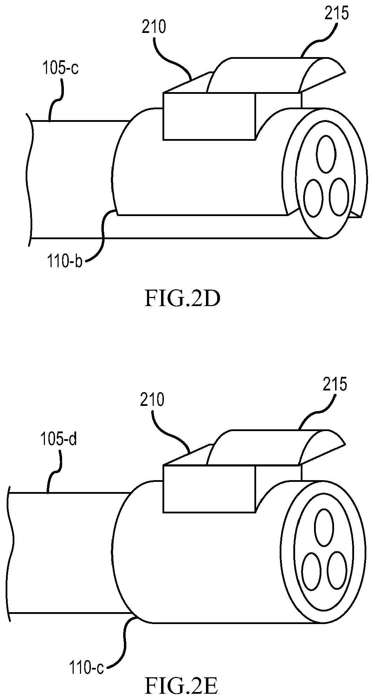

With reference to **FIG 2D** and/or **FIG. 2E**, an operative element 215 may be coupled with the support member 210. In such embodiments, the therapeutic or diagnostic device may be used to move the operative element 215 to a target treatment area. As shown in FIGs. 2D and/or 2E, the operative element 215 may extend off of the support member 210. In some embodiments, no portion of the operative element 215 extends over the edges of the support member 210.

**FIG. 3A** is a perspective view of a releasable mount device 110-d according to various embodiments. The releasable mount device 110-d may include a tubular base 300 and a support member 210-a. The tubular base 300 may have longitudinal edges 305 that extend from the distal end 320 to the proximal end 325. **FIG. 3B** is a cross-sectional view of the distal end 320 to the proximal end 325 of the releasable mount device 110-2 of FIG. 3A. **FIG. 3C** is a perspective bottom view of the interior surface 330 of the releasable mount device 110-d of FIG. 3A. The releasable mount device 110-d may removably attach anywhere along the length of a therapeutic or diagnostic device. The releasable mount device 110-d may be configured to be removed without damaging the releasable mount device 110-d or the therapeutic or diagnostic device it is attached to. In various embodiments, an operative element (not shown) may be mounted to the support member 210-a and used in conjunction with the therapeutic or diagnostic device. After being attached to a therapeutic or diagnostic device, the releasable mount device 110-d may be removed from the therapeutic or diagnostic device using one or more features of the tubular base 300, such as a contact member 335 and a slot 340.

The tubular base 300 may include an interior surface 330, an exterior surface 345, a distal end 320 and a proximal end 325. In various embodiments, the tubular base 300 may be formed of a thermoplastic or thermoset polymer. The tubular base 300 may be formed of various materials including, but not limited to, acrylonitrile butadiene styrene (ABS), silicone, PEBAX®, urethane, or isoprene. The inner surface 330 of the tubular base 300 may be shaped to engage a therapeutic of diagnostic device. For example, the interior surface 330 may have a hemispherical, semi-circular or other shape which may or may not correspond to an outer surface of the therapeutic of diagnostic device. In various embodiments, the shape of the interior surface 330 may be smaller than a similarly shaped (i.e., hemispherical or semi-circular) therapeutic or diagnostic device to provide for a snug fit between the releasable mount device 110-d and the therapeutic or diagnostic device. The snug fit may help ensure that the releasable mount device 110-d is securely attached to the therapeutic or diagnostic device. In some embodiments, the tubular base 300 may be formed from a non-elastomeric material, such as a non-rubber material or a non-silicone material.

The snug fit may be related to a clamping force exerted by the releasable mount device 110-d on the therapeutic or diagnostic device. In various embodiments, the clamping force relates to an allowable amount of relative movement between the releasable mount device 110-d about an x, y and/or z axis and the device to which it is attached. The relative movement may be rotational movement about the x, y, and/or z axis or translational (axial) movement.

Similarly, the clamping force may include a rotational force about an x, y, and/or z axis. The clamping force may also include an axial force about an x, y, and/or z axis. In various embodiments, the clamping force is sufficient to limit or reduce movement of the releasable mount device 110-d in at least one direction. In various embodiments, the clamping force is sufficient to limit or reduce movement of the releasable mount device 110-d in two or three directions. In various embodiments, the clamping force is sufficient to rotate the releasable mount device 110-d a distance of 0.2 inch with a tangential force equal to or greater than 0.8 lb-force (when the therapeutic or diagnostic device is dry) and 0.5 lb-force (when the therapeutic or diagnostic device is lubricated). In various embodiments, the clamping force is sufficient to rotate the releasable mount device 110-d a distance of 2% of the diameter of the therapeutic or diagnostic device. In various embodiments, the axial force required to detach the releasable mount device 110-d from the therapeutic or diagnostic device is greater than or equal to 1.0 lb-force. One may appreciate that from the description herein that the clamping force may be designed or selected based on the functional requirements of the therapeutic or diagnostic device. For example, the clamping force may be selected to be in a range to adequately secure the releasable mount device 110-d to the therapeutic or diagnostic device during treatment while still allowing a user to remove the releasable mount device 110-d from the therapeutic or diagnostic device without specialized tools.

The interior surface 330 of the tubular base 300 may include one or more friction members 350. In various embodiments, each friction member 350 may have an outer surface which extends radially inwardly. The outer surface of the friction member 350 may engage an outer surface of a therapeutic or diagnostic device on which the releasable mount device 110-d is mounted to prevent the releasable mount device 110-d from changing positions with respect to the therapeutic or diagnostic device. As shown in FIGs. 3A-3C, the friction member 350 may extend in a longitudinal direction along the interior surface 330 of the tubular base 300. The friction member 350 may be compressible and configured to apply a restoring force under compression. In various embodiments, the friction member 350 is implemented as a compressible foam, a low durometer pad, a tacky adhesive, or a combination of the same. In various embodiments, the friction member 350 covers a portion or the entire interior surface 330 of the tubular base 300.

The exterior surface 345 of the tubular base 300 may have a hemispherical, semi-circular or other shape which may or may not correspond to an outer surface of the therapeutic or diagnostic device. The exterior surface 345 may have a smooth texture and is in contact with a support member 210-a. The distance between the exterior surface 345 and the interior surface 330 may form the thickness of the tubular base 300. In some embodiments, the thickness of the tubular base 300 is constant, while in other embodiments, the thickness of the tubular base 300 varies.

In various embodiments, the tubular base 300 may include one or more longitudinal edges 305 that extend from the distal end 320 to the proximal end 325. In some embodiments, the tubular base will include two longitudinal edges 305, with the two longitudinal edges 305 being on either side of the slot 340. The longitudinal edges 305 of the tubular base 300 may be straight and extend substantially parallel to a longitudinal axis of the tubular base 300. Each of the longitudinal edges 305 may comprise a contact member 335 along the entire longitudinal length. The contact members 335 may extend away from the interior surface 330 of the tubular base 330 to form a radiused contact surface. For example, the contact members 335 in FIGs. 3A-3C have a rounded, curviplanar shape.

A contact member 335 may come into contact with the outer surface of the therapeutic or diagnostic device while attaching the releasable mount device 300 to the therapeutic or diagnostic device and after the releasable mount device 110-2 is completely attached to the therapeutic or diagnostic device. The radiused contact surface of the contact member 335 may prevent the longitudinal edges 305 from penetrating and damaging the outer surface of the therapeutic or diagnostic device when the releasable mount device 110-d is attached to the therapeutic or diagnostic device. The radiused contact surface of the contact member 335 may also aid in removal of the releasable mount device 110-d from the therapeutic or diagnostic device by providing an increased surface area for applying a removal force to a contact member 335. The longitudinal edges 305 of the tubular base 300 may also be configured to deflect in response to a contact force, for example when the releasable mount device 110-d is transversely attached to a therapeutic or diagnostic device.

The longitudinal edges 305 of the tubular base 300 may define a slot 340. The slot 340 of the tubular base 300 is an opening that extends along the longitudinal edges 305 between the distal end 320 and proximal end 325 of the tubular base 300. The slot 340 may be used to attach the releasable mount device 110-d to the therapeutic or diagnostic device in a transverse direction with respect to the therapeutic or diagnostic device.

The slot 340 may have a slot width that changes widths. For example, the slot width may expand and/or retract as the tubular base 300 of the releasable mount device 110-d is attached to or removed from the therapeutic or diagnostic device. In various embodiments, the expansion of the tubular base 300 may be entirely within an elastic range. In various embodiments, the tubular base 300 is constructed of a superelastic material and the material undergoes changes entirely in the elastic range when it is attached to and removed from the therapeutic or diagnostic device. In various embodiments, the tubular base 300 is constructed of a shape memory material (e.g., nitinol). The tubular base 300 and manufacturing process may make use of the superelastic and/or shape memory properties of the material. For example, the tubular base 300 may be heated after it is attached to the therapeutic or diagnostic device so it conforms to the therapeutic or diagnostic device to which it is attached. The tubular base 300 may have a transition temperature and shape selected so the clamping force increases when the tubular base 300 is heated within the body.

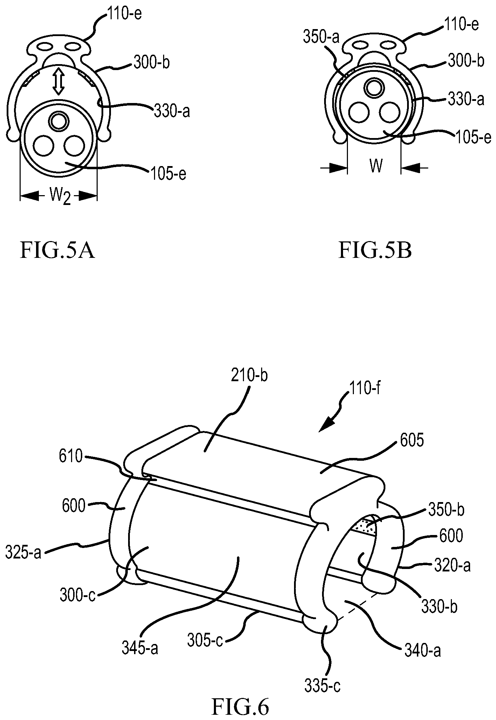

The width of the slot 340 may defined as the distance between the two longitudinal edges 305 and/or the two contact members 345. In some embodiments, the width of the slot 340 is less than the width of the therapeutic or diagnostic device to which it is attached. The slot 340 may have a first width or dimension when the releasable mount device 110 -d is not attached or in contact with the therapeutic or diagnostic device. The releasable mount device 110-d may be attached to the therapeutic or diagnostic device by placing the slot 340 over a portion of the outer surface of the therapeutic or diagnostic device. The portion may be located at the distal end of the therapeutic or diagnostic device along the longitudinal axis in a direction transverse to the therapeutic or diagnostic device. As the releasable mount device 110-d is attached to the therapeutic or diagnostic device, the width of the slot 340 may expand to a second width. The second width may be as large as the diameter of the therapeutic or diagnostic device to which the releasable mount device 110-d is attached. The width of slot 340 may have a third width when the releasable mount device 110-d is completely attached to the therapeutic or diagnostic device. The third width may be greater than the first width, for example when the interior surface 330 of the releasable mount device 110-d has a shape that is smaller than the outer surface of the therapeutic or diagnostic device. The third width may be smaller than the second width in order to help ensure a snug fit between the releasable mount device 110-d and the therapeutic or diagnostic device. Slot widths are discussed in more detail with respect to FIGs. 5A and 5B.

In various embodiments, the tubular base 300 includes a spring fastener (not shown). The spring fastener may extend across the slot 340 between each of the longitudinal edges 305. The spring fastener may be a planar spring, an elastomer, or a textile.

The releasable mount device 110-d may include a support member 210-a that is coupled with the tubular base 300. In various embodiments, the support member 210-a is rigid and may be positioned opposite the slot 340 on the exterior surface 345 of the tubular base 300. The support member 210-a may include a ridge 355, and a foot portion 360. The foot portion 360 may include an upper foot 365 and a lower foot 370. In some embodiments, the support member 210-a is arranged along a longitudinal direction and extends between the distal end 320 and the proximal end 325 of the exterior surface 345 of tubular base 300. In some embodiments, the foot portion 360 is located at the distal end of the tubular base 300 and the ridge portion extends from the foot portion 360 to the distal end 320 of the tubular base 300. In various embodiments, the support member 210-a (including the ridge 355 and the foot portion 360) and the tubular base 300 are monolithically formed. The outer surfaces of the ridge 355, the upper foot 365, and the lower foot 370 may be smooth. In various embodiments, the support member 210-a and the tubular base 300 are formed separately.

When formed separately, the support member 210-a may be pivotably coupled to the tubular base 300. A pivot coupling structure (not shown) may form an attachment between the support member 210-a and the tubular base 300 in various positions between the support member 210-a and the tubular base 300, including but not limited to the distal end, the proximal end, or the center of the support member 210-a. In various embodiments, the support member 210-a may be positioned such that the proximal end of the support member 210-a is pivotably coupled to the distal end 320 of the tubular base 300 allowing the distal end of the support member 210-a to extend past the distal end 320 of the tubular base 300. Examples of a pivot coupling structures, for example a rotational support, are described in more detail in U.S. Patent Pub. Nos. 2007/0118104 to Wallace et al., and 2009/0177194 to Wallace et al., the disclosures of which are incorporated herein by reference for all purposes.

The ridge 355 may have an elevated portion having a semi-cylindrical body. The shape of the semi-cylindrical body may comprise half a cylinder divided along the longitudinal axis of the cylinder. The semi-cylindrical body may extend in a longitudinal direction along the exterior surface 345 of the tubular base 300 between the foot portion 360 and the distal end 320of the tubular base 300. The distal end of the ridge 355 may gradually curve from the top of the ridge 355 to the exterior surface 345 of the tubular base 300. The ridge 365 may include one or more apertures (not shown) that allow wires or other components to pass through the ridge 365. The apertures may run longitudinally or transversely through the ridge 365. The wires extending through the apertures in the ridge 365 may be conveniently placed near a mounted operative element while being protected by the structure of the ridge 365. For example, the apertures may be used to route the wires of an operative element coupled with the support member 210-a from the operative element back towards the proximal end 325 of the tubular base 300 and towards the therapeutic or diagnostic device. In various embodiments, the ridge 355 has a shape other than a semi-cylindrical shape, such as a square, pentagonal, oval or other shape.

The ridge 355 may provide a keying structure that provides for coupling between the ridge 355 and an operative element. The keying structure may maintain a position of a mounted operative element on the support member 210-a. For example, when mounted over the ridge 355, the shape of the ridge 355 may provide lateral stability for the mounted operative element. In some embodiments, the operative element includes a recess configured to receive the ridge 355 of the support member 210-a.

The upper foot 365 and lower foot 370 may be positioned along the longitudinal axis of the tubular base 300 near or at the proximal end 325 of the tubular base 300. The upper foot 365 may include a top surface, a bottom surface, two side surfaces, a distal end, and a proximal end. The distal end of the upper foot 365 may extend wider than the proximal end of the ridge 355. The upper foot 365 may include one or more apertures 375. The apertures 375 of the upper foot 365 may be similar to the apertures of the ridge 355 and may be used to route the wires of an operative element coupled with the support member 210-a. The top surface of the upper foot 365 may have a curviplanar shape corresponding to an inner surface of a body lumen. The upper foot 365 may provide protection of an operative element during retraction. The upper foot 365 may be slightly taller than the operative element when the operative element is in its home position (flat against the mount). During retraction, this may prevent the operative element from being caught and lifted from the back end, which could make removal difficult. Some configurations may prevent high retraction forces and potential tissue damage. The operative element may be configured to pivot relative to the base in cases, which may lead to issues with retraction..Each of the two side surfaces may also have a curviplanar shape that extends between the top surface of the lower foot 370 and the top surface of the upper foot 365.

A portion of the distal end of the lower foot 370 may extend wider than the proximal end of the ridge 355 and wider than the distal end and proximal end of the upper foot 365. The lower foot 370 may include one or more apertures (not shown). The apertures of the lower foot 370 may be similar to the apertures of the ridge 355 and the apertures 375 of upper foot 365, and may be used to route the wires of an operative element. The top surface of the lower foot 370 may have a curviplanar shape that corresponds to the shape of the bottom surface of the upper foot 365. In various embodiments, both the upper foot 365 and lower foot 370 have a shape other than a curviplanar shape, such as for example a cubic, spherical, pyramidal, or other shape.

The support member 210-a may be used to couple an operative element with the releasable mount device 110-d. In various embodiments, the operative element may be coupled with the distal end of the support member 210-a in a fixed position. The operative element may be mounted to the ridge 355 and positioned flush against the foot portion 360. By positioning the operative element flush against the foot portion 360, the operative element may be secured in place and prevented from sliding along the ridge 355. In various embodiments, the operative element may be pivotably coupled with the support member 210-a.

Returning to FIG. 3B, the cross-sectional view of the releasable mount device 110-d including the tubular base 300 and the support member 210-a of FIG. 3A is shown in accordance with various embodiments. The tubular base 300 as shown in FIG. 3B may include contact members 335, friction members 350, and a slot 340. The interior surface 330 of the tubular base 300 may form a hemispherical or semi-circular shape with a radius of curvature "R₁". The radius of curvature "R₁" may change widths, for example increase and decrease, as the releasable mount device 110-d is mounted on the therapeutic or diagnostic device, such as for example an endoscope. The radius of curvature "R₁" of the interior surface 330 may be less than the curvature radius of an outer surface of a therapeutic or diagnostic. The radius of curvature "R₁" may have a first length or dimension when the releasable mount device 110-d is not mounted or in contact with the therapeutic or diagnostic device. The slot 340 may have a first width "W₁," which may be the width of the slot 340 when the releasable mount device 110-d is not in use or being mounted on a therapeutic or diagnostic device.

The releasable mount device 110-d may be mounted on the therapeutic or diagnostic device by placing the slot 340 over the therapeutic or diagnostic device. The slot 340 is placed over the outer surface of the therapeutic or diagnostic device and along the longitudinal axis in a direction transverse to the therapeutic or diagnostic device. As the releasable mount device 110-d is placed over the therapeutic or diagnostic device, the length of the radius may increase to a second width. The second width may be as large as the radius of the outer surface of the therapeutic or diagnostic device to which the releasable mount device 110-d is attached. The radius length may have a third width when the releasable mount device 110-d is completely mounted on the therapeutic or diagnostic device. The third width of the radius length may be greater than the first width of the radius length, for example when the interior surface 330 of the releasable mount device 110-d has a shape that is smaller than the outer surface of the therapeutic or diagnostic device. The third width of the radius length may be smaller than the second radius length width in order to help ensure a snug fit between the releasable mount device 110-d and the therapeutic or diagnostic device.

The thickness of the tubular base 300 (i.e., the distance between the exterior surface 345 and the interior surface 330) may be a uniform thickness as shown in FIGs. 3A-3C. In such embodiments, "t1" proximate the slot 340 is equal "t2" proximate the support member 210-a. In various embodiments, the thickness of the tubular base 300 is non-uniform. For example, the portion of the tubular base 300 proximate "t1" may be thinner or thicker than the portion of the tubular base 300 proximate "t2".

FIG. 3C is a perspective bottom view of the interior surface 330 of the releasable mount device 110-d. Attached to the interior surface 110 may be friction members 350. The friction members 350 may extend in a longitudinal direction between the distal end 320 and the proximal end 325 of the releasable mount device 110-d. The friction members 350 of FIG. 3C may have a uniform width and may be approximately equal in length. In various embodiments, friction members attached to the inner surface 330 of the tubular base 300 may extend in a direction other than a longitudinal direction, have a non-uniform width and shape, and/or may differ in shape and direction from each other.

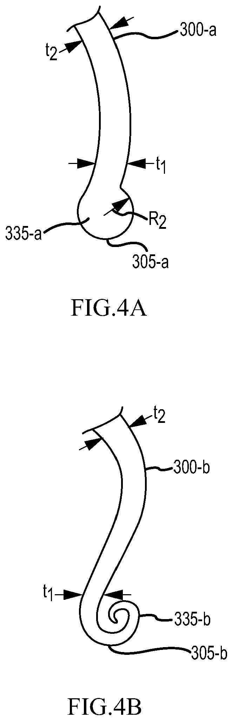

**FIG. 4A** is an enlarged cross-sectional view of a longitudinal edge 305-a having a curviplanar shape according to various embodiments. The longitudinal edge 305-a may include a contact member 335-a along the entire length of the longitudinal edge 305-a. The longitudinal edge 305-a and contact member 335-a of FIG. 4A are similar to those described in FIGs. 3A-3C. The contact member 335-a may come into contact with the sheath of a therapeutic or diagnostic device and may extend away from an interior surface 330 of the tubular base 300 to form a radiused contact surface.

The contact member 335-a may prevent the longitudinal edge 305-a from penetrating and damaging the outer sheath of the therapeutic or diagnostic device. In general, a contact member, such as contract member 335-a, may be smooth and sized to provide a smooth transition between the releasable mount device and a therapeutic or diagnostic device. During insertion, if the contact member is too large, it may cause tissue damage. A gripping force may be applied by the contact member 335-a to secure the releasable mount device in place. In various embodiments, the contact member 335-a has a curviplanar shape with radius "R₂" and a smooth outer surface as shown in FIG. 4A. In various embodiments, the contact member 335-a may have any of a variety of cross-sectional shapes, including a cubic, spherical, pyramidal, or other shape. The releasable mount device may be easily removed by providing an increased surface area to exert a removing force against the releasable mount device. The tubular base 300-a shown in FIG. 6A may have a uniform thickness, in which case "t1" is equal "t2." In various embodiments, the thickness "t1" of the tubular base 300-a proximate the longitudinal edge 305-a is about 0.03 inch and a thickness "t2" of the tubular base 300-a proximate the support member is about 0.06 inch. In various embodiments, the tubular base 300-a proximate the longitudinal edge 305-a has a thickness in the range of between about 0.005 inch and about 0.1 inch.

**FIG. 4B** is an enlarged cross-sectional view of a longitudinal edge 305-b having a serif shape according to various embodiments. In various embodiments, each of the longitudinal edges 305-b of FIG. 4B comprises a serif shape along their entire longitudinal length. The contact members 335-b may extend back toward the interior of the tubular base 300 at the distal end as shown in FIG. 4B. The tubular base 300-b proximate the longitudinal edge 305-b may be thinner than the tubular base 300-b proximate the support member. For example, the thickness "t1" may be less than the thickness "t2". In various embodiments, the thickness "t1" may be greater than, less than, or about equal to the thickness "t2". In various embodiments, the tubular base 300-b proximate the longitudinal edge 305-b may have a thickness in the range of between about 0.03 inch and about 0.005inch.

**FIG. 5A** is a distal end view of a releasable mount device 110-e wherein the slot has a second width "W₂" according to various embodiments. When the releasable mount device 110-e is partially mounted on a therapeutic or diagnostic device 105-e, the slot of releasable mount device 110-e may have a second width "W₂". The width "W₂" is the width of the slot when the releasable mount device 110-e is mounted partially over a therapeutic or diagnostic device 105-e. When the slot has a width "W₂", the radius of curvature of the interior surface 330-a is greater than the radius of curvature of the inner surface when the slot has a first width "W₁". The larger radius of curvature is due to the radius of curvature of the outer surface of the therapeutic or diagnostic device 105-e being larger than the radius of curvature of the interior surface 330-a of the releasable mount device 110-e.

**FIG. 5B** is distal end view of a releasable mount device 110-e wherein the slot has a third width "W₃" according to various embodiments. The releasable mount device 110-e having a slot may have a third width "W₃" when the releasable mount device 110-e is completely mounted on the therapeutic or diagnostic device. When completely mounted on releasable mount device 110-e, the outer surface of the therapeutic or diagnostic device may be in contact with either a portion of the interior surface 330-a of the tubular base 300-b or one or more friction members 350-a. The third width "W₃" may be essentially equal to the distal end diameter of the therapeutic or diagnostic device wherein the slot width decreases from the second width "W₂" to a third width "W₃." An inner diameter of the tubular base 300-b in a non-expanded state may be essentially equal to the second width "W₂."

In various embodiments, the first width "W1" is in the range of about 2mm to about 20mm. In various embodiments, the second width "W1" is selected from the group consisting of about 2mm, about 3mm, about 4 mm, about 5mm, about 6 mm, about 7mm, about 8mm, about 9mm, about 10mm, about 11mm, about 12 mm, about 13mm, about 14mm, about 15mm, about 16mm, about 17, about 18 mm, about 19mm, about 20mm, about 21mm, about 22 mm, about 23, about 24, or about 25 mm. In various embodiments, the second width "W2" is selected from the group consisting of about 1.8mm, about 2.0mm, about 2.8mm, about 3.1mm, about 3.2mm, about 3.4mm, about 3.8mm, about 3.9mm, about 4.0mm, about 4.1mm, about 4.8mm, about 4.9mm, about 5.1 mm, about 5.3 mm, about 5.5mm, about 5.9mm, about 6.9mm, about 8.8mm, about 9.2mm, about 9.8mm, about 9.9mm, about 10.9mm, about 11.3mm, about 11.7mm, about 12.0mm, about 12.7mm, about 12.8mm, about 12.9mm, about 13.2mm, about 13.7mm, about 13,8mm, about 13.9mm, about 14.2mm, about 14.6mm, or about 15.0mm.. In various embodiments, the second width "W2" is in the range between about 8.6 to about 13.8 mm.

**FIG. 6** is a perspective view of a releasable mount device 110-f including a protective guard 600 in accordance with various embodiments. The releasable mount device 110-f includes a tubular base 300-c and a support member 210-b. The tubular base 300-c is similar to the tubular base 300 of FIGs. 3A-3C and includes an interior surface 330-b, exterior surface 345-a, longitudinal edges 305-c, slot 340-a, and contact members 335-c. One or more friction members 350-b may be positioned on the interior surface 330-b. Support member 210-b may include an upper surface 605 and side surfaces 610. The upper surface 605 and side surfaces 610 may extend along a longitudinal length of the releasable mount device 110-f between a distal end 320-a and a proximal end 325-a. The upper surface 605 may be smooth, rigid, and substantially flat as illustrated in FIG. 6. The shape of the upper surface 605 may be curved, cubic, spherical, pyramidal, or other shape. The side surfaces 610 may extend from the top surface 605 to the exterior surface 345-a of the tubular base 300-c. In various embodiments, an operative element may be attached along the entire longitudinal plane between the distal end and proximal end of the support member 210-b. In various embodiments, the tubular base 300-c and the support member 210-b may be monolithically formed.

The releasable mount device 110-f may include one or more protective guards 600. For example, the protective guard 600 may be located on the distal end 320-a of the tubular base 300-c, the proximal end 325-a of the tubular base 300-c, or both as illustrated in FIG. 6. In various embodiments, a protective guard 600 may be positioned over the structures of the distal end 320-a and/or proximal end 325-a of the tubular base 300-c and support member 210-b. In various embodiments, the structures include the top surface 605, side surfaces 610, exterior surface 345-a, inner surface 330-b, and contact members 335-c. The protective guard 600 may act to prevent damage to the outer sheath of a therapeutic or diagnostic device that comes into contact with the tubular base 300-c. The protective guard 600 may provide rotational stability when the releasable mount device 110-f is attached to the therapeutic or diagnostic device. In various embodiments, the protective guard 600 may be constructed from a material including, but not limited to, acrylonitrile butadiene styrene (ABS), silicone, PEBAX®, urethane, or isoprene.

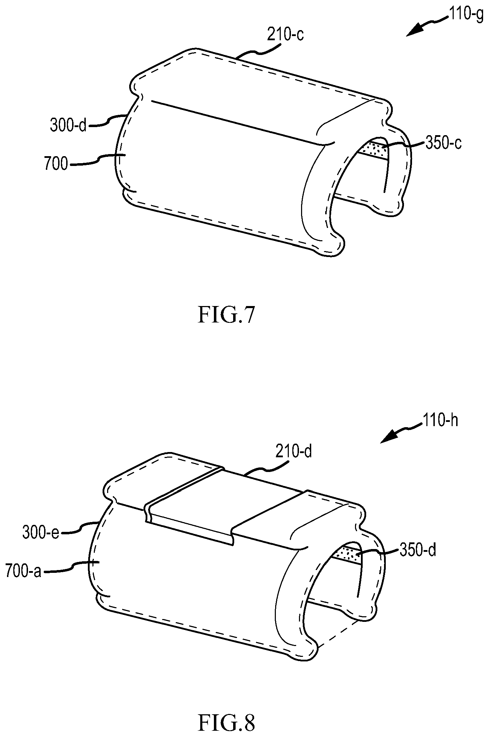

With reference to **FIG. 7**, the releasable mount device 110-g may include an exterior coating 700 in accordance with various embodiments. The releasable mount device 110-g of FIG. 7 may include a support member 210-c, tubular base 300-d, and friction members 350-c similar to the releasable mount device 110-f of FIG. 6. The releasable mount device 110-g of FIG. 7 differs from the releasable mount device 110-f of FIG. 6 in that an exterior coating 700 may be applied to the exterior surface (not shown) of the releasable mount device 110-g of FIG. 7. In various embodiments, the exterior coating 700 may be applied to the outer surface, the inner surface, or both of the releasable mount device 110-g, including the outer surface of tubular base 300-d and the support member 210-c. The exterior coating 700 may provide a barrier to protect the outer sheath of the therapeutic or diagnostic device that comes into contact with the tubular base 300-d.

The exterior coating 700 may be a gel, polymer, bioactive agent or other material. In various embodiments, the support member 210-c is coated with a biomolecule such as a pharmaceutical agent, nucleic acid, amino acid, sugar, or lipid. In various embodiments, the support member 210-c is coated with an antihyperplastic agent such as an antithrombotic agent (e.g. heparin), an antiplatelet agents (e.g. aspirin, arachidonic acid, and prostacyclin), or an antibody to platelet-derived growth factors. Other suitable biological coating materials and additives include endothelial cells, stem cells, and cell growth factors. In various embodiments, the operative element that may be coupled with the support member 210-c is coated with a biocompatible plastic such as polytetrafluoroethylene (PTFE), expanded PTFE (ePTFE), polypropylene, or poly(lactide).

Friction members 350-c may be used in conjunction with exterior coating 700. In various embodiments, the friction members 350-c may be positioned on top of the exterior coating 700. In various embodiments, the exterior coating 700 may be applied to encase the friction members 350-c.

With reference to **FIG. 8****,** the exterior coating 700-a may not encase the entire releasable mount device 110-h in accordance with various embodiments. FIG. 8 is a perspective view of a releasable mount device 110-h including a partial exterior coating 700-a. The releasable mount device 110-h of FIG. 8 may include a support member 210-d, tubular base 300-e, and friction members 350-d similar to the releasable mount device 110-f, 110-g of FIGs. 6 and 7, respectively. The exterior coating 700-a shown in FIG. 8 may cover the entire tubular base 300-e but does not cover a portion of the surface of support member 210-d. In various embodiments, one or more portions of the exterior coating 700-a may be removed to expose a surface of the support member 210-d. The portion of exterior coating 700-a removed may be located anywhere on the releasable mount device 110-h.

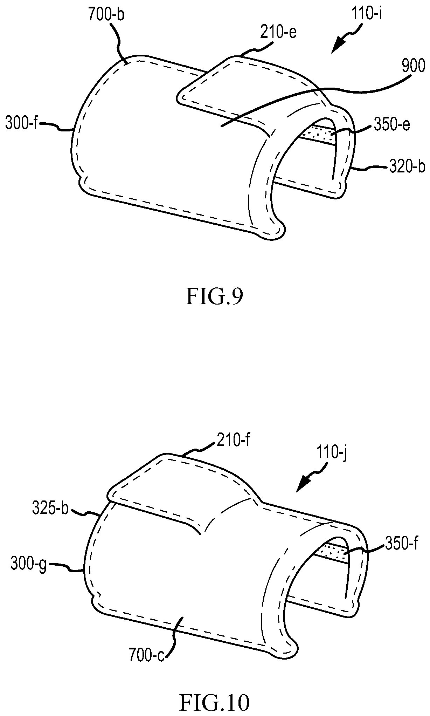

FIGs. 9-11 illustrate embodiments of a releasable mount device with an exterior coating. One will appreciate from the description herein that the exterior coating may be optional. In various embodiments, the releasable mount devices shown may include a partial coating or no coating.

**FIG. 9** is a perspective view of a releasable mount device 110-i including a support member 210-e, a tubular base 300-f, friction members 350-e, and an exterior coating 700-b in accordance with various embodiments. The tubular base 300-f, friction members 350-e, and exterior coating 700-b are similar to that of FIGs. 6-8. The support member 210-e in FIG. 9 differs in structure, size, and position from FIGs. 6-8. In various embodiments, the support member 210-e may extend from the distal 320-b end of the tubular base 300-f to the mid-section of the tubular base 300-f. The support member 210-e may be planar and may have a ramped leading edge at the distal end of the support member 210-e. The ramped leading edge may be less traumatic when inserted into an inner lumen of the body. The support member 210-e may include a base member 900 that is positioned underneath the support member 210-e and extends from the distal end of the support member 210-e to the proximal end of the support member 210-e. The base member 900 may have various shapes including but not limited to a cubic, a cylindrical, or other shape. In various embodiments, the side surfaces of the base member 900 may be curved or straight and extend from the tubular base 300-f to the bottom of the support member 210-e.

**FIG. 10** is a perspective view of a releasable device mount 110-j according to various embodiments that may include a support member 210-f at a proximal end 325-b, a tubular base 300-g, friction members 350-f, and an exterior coating 700-c. The tubular base 300-g, friction members 350-f, and exterior coating 700-c are similar to that of FIGs. 6-9. The support member 210-f is similar to that described with respect to FIG. 9 except for the position of the support member 210-f. The support member 210-f in FIG. 10 may include a ramped leading edge and extends from the mid-section of the tubular base 300-g to the proximal end 325-b of the tubular base 300-g. The varying position of the support member may allow more precise, targeted treatment to various tissue sites.

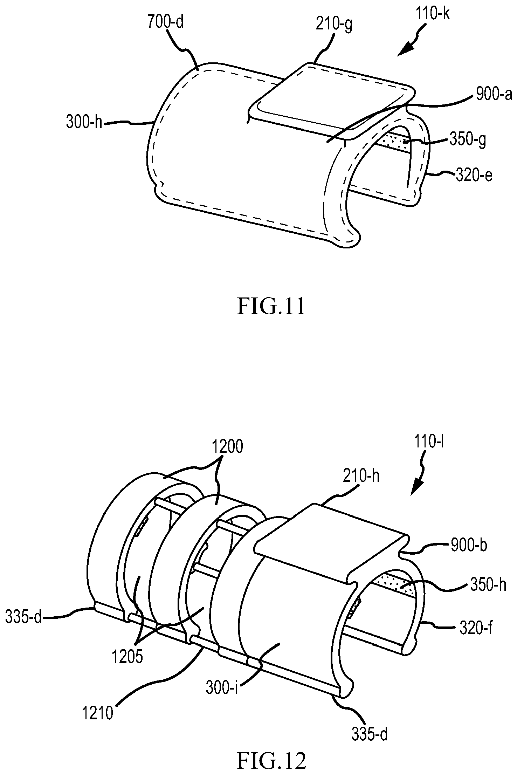

**FIG.** 11 is a perspective view of a releasable mount device 110-k according to various embodiments that may include a support member 210-g at a distal end 320-c, a tubular base 300-h, friction members 350-g, and an exterior coating 700-d. The tubular base 300-h, friction members 350-g, and exterior coating 700-d are similar to that of FIGs. 6-10. The support member 210-g is similar to that described with respect to FIG. 9 except the support member 210-g lacks the ramped leading edge on the distal end of the support member 210-g. The support member 210-g in FIG. 11 may extend from the distal end 320-c of the tubular base 300-h to the mid-section of the tubular base 300-h and is essentially planar. As in FIG. 9 and 10, the support member 210-g may be elevated on a base member 900-a.

**FIG. 12** is a perspective view of a releasable mount device 110-l in accordance with various embodiments. The releasable mount device 110-l may include a slotted tubular base 300-i, a support member 210-h, and friction members 350-i. The support member 210-h, tubular base 300-i, and friction members 350-i are similar to those described in FIGs. 6-11 except that the tubular base 300-i in FIG. 12 may include at least one lateral piece 1200 formed by at least one lateral slot 1205 that extends between the longitudinal edges of the tubular base 300-i. The slot 1205 is formed between the pieces 1200 of the tubular base 300-i. The contact members 350-h of the tubular base 300-i may also differ from FIGs. 6-11 in that a rod 1210 may extend through the contact members 335-d of the tubular pieces 1205 and the slot 1205 along the longitudinal axis. In various embodiments, the tubular pieces 1205 may be fixed or may be allowed to slide along the rod 1210 along the longitudinal axis formed by the rods 1210. The rods 1210 may be positioned anywhere along the tubular base 300-i. The support member 210-h of FIG. 12 may be similar to the support member 210-g of FIG. 11 in that it is essentially planar, positioned at the distal end 320-f of the tubular base 300-i, and is attached to a base member 900-b. In various embodiments, the distal end of the support member 210-g may be flush with the distal end 320-f of the tubular base 300-i.

The releasable mount device 110-1 of FIG. 12 may have a lower clamping force than the releasable mount devices described in FIGs. 6-11. The lower clamping force may result from having a slotted tubular base 300-i rather than a solid tubular base. In some embodiments, one or more section of the multi-piece releasable mount device might have different clamping forces. For example, more distal portions may clamp more than portions more proximal, which may depend on the needs of the releasable mount device. In some cases, high forces may be required distally, but only low supportive forces may be required proximally.

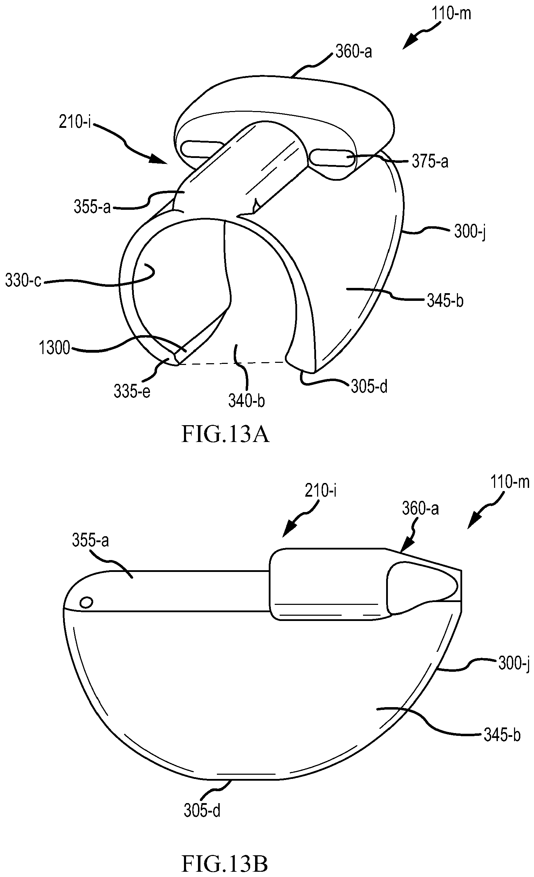

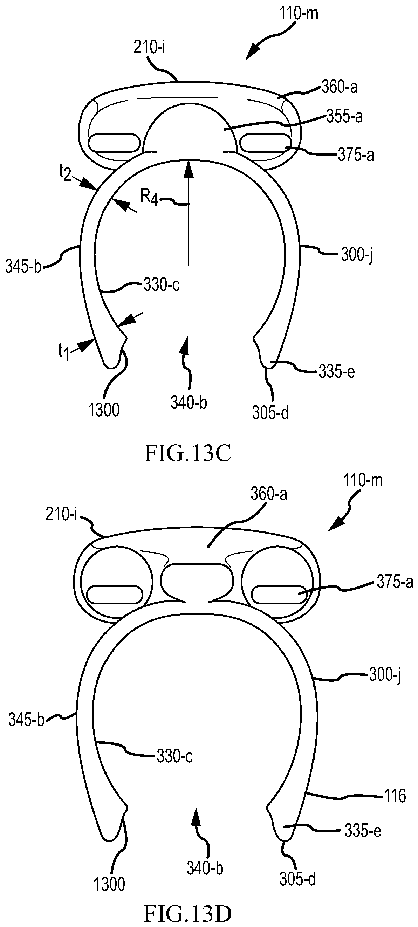

**FIG. 13A, FIG. 13B****,** **FIG. 13C****,** and/or **FIG. 13D** illustrate various embodiments of a releasable mount device 110-m including a tubular base 300-j and a support member 210-i. The tubular base 300-j may include longitudinal edges 305-d, contact members 335-e, a groove 1300, and a slot 340-b. The releasable mount device 110-m as illustrated does not include a friction member or gripping member, though either may be included within the releasable mount device 110-m of FIGs. 13A-13D. The support member 210-i may include a ridge 355-a, a foot portion 360-a, and apertures 375-a. Support member 210-i may be similar to the support member of FIGs. 3A-3C. The tubular base 300-j may be similar to the tubular base of FIGs. 3A-3C except for the shape and the presence of grooves 1300 in the contact member 335-e.

The length of tubular base 300-j along longitudinal edges 305-d may be shorter than the length of tubular base 300-j proximate the support member 210-i. In various embodiments, the tubular base 300-j may taper in length from the area proximate the support member 210-i to the longitudinal edges 305-d to form a curved shape. For example, the tubular base 300-j may have a scalloped shape.

The contact member 335-e may include a groove 1300 that extends along all or a portion of the contact member 335-e in the longitudinal direction. The groove 1300 may create an inward concave shape on the upper portion of the contact member 335-e. The radius of curvature of a lower portion of the contact member 335-e may be less than a corresponding radius of curvature for the contact member 335 shown in FIGs. 3A-3C.

The tubular base 300-j may have varying thickness. The varying thickness may be due to the interior surface 330-c having a radius of curvature shorter than the radius of curvature of the exterior surface 345-b. As a result, the thickness "t1" of tubular base 300-j proximate the longitudinal edges 305-d may be greater than thickness "t2" of the tubular base 300-j proximate the support member 210-i.

In various embodiments, radius "R4" labeled in FIG13C may be smaller than the radius of the interior surface 330-c of the tubular base 300-j. In various embodiments, R4 may allow the shape of the releasable mount device 110-m to change between a horseshoe shape when it is off the therapeutic or diagnostic device, to a rounder shape when it is on the therapeutic or diagnostic device. When the design of the releasable mount device 110-m changes from a horseshoe shape to a round shape, a spring force may be generated from the deflection of the tubular base 300-j. The deflection of the tubular base 300-j may secure the tubular base 300-j to the therapeutic or diagnostic device.

The slot 340-b of FIGs. 13A-13D may be formed between contact members 335-e. The slot 340-b may have different width based on contact with a therapeutic or diagnostic device. In various embodiments, the slot 340-b may have widths of a first width "W1" when not in contact with an endoscope, a second width "W2" when partially mounted over a therapeutic or diagnostic device, and a third width "W3" when completely mounted over a therapeutic or diagnostic device. The details of the first, second and third widths of slot 340-b of FIGs. 13A-13D are similar to the widths discussed with respect to FIGs. 3A-3C.

Some embodiments may utilize nest tubular bases (not shown). This may allow for a releasable mount device to fit therapeutic or diagnostic devices with different diameters. If the therapeutic or diagnostic device is small, for example, two next tubular bases may be utilized. For a therapeutic or diagnostic device with a larger diameter, the inner tubular base could be removed to allow the outer tubular base with a larger diameter to accommodate a therapeutic or diagnostic device with a large diameter. The other features of a releasable mount device (e.g., support member) may only be coupled with the outer tubular base, while the inner tubular base may just be to make the diameter smaller.

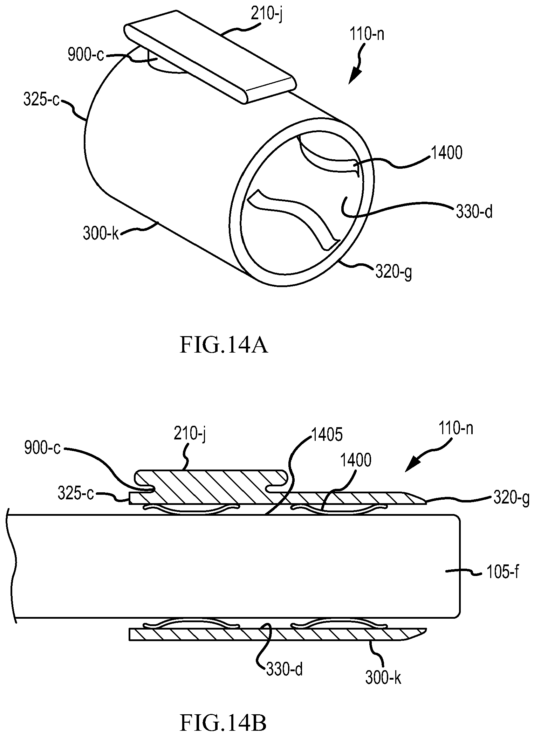

**FIG. 14A** is a perspective view of the releasable mount device 110-n according to various embodiments. The releasable mount device 110-n may have no slot and a gripping member 1400 including a spring. FIG. 14B is a cross-sectional side view of the releasable mount device 110-n shown in FIG. 14A. The releasable mount device 110-n of FIGs. 14A and 14B may include a tubular base 300-k, a support member 210-j, a gripping member 1400, and a gap 1405.

The tubular base 300-k of the releasable mount device 110-n of FIGs. 14A and14B may have a solid body forming a continuous circumference, thereby not forming a slot. The tubular base 300-k may have a distal end 320-g, a proximal end 325-c, and a central longitudinal axis for mounting to a distal end of a therapeutic or diagnostic device 105-f. The tubular base 300-k may have a diameter which is larger than the diameter of the distal end of the therapeutic or diagnostic device 105-f. For example, the tubular base 300-k may have a diameter of about 4mm, about 12mm, about 18mm, about 22mm, or about 25mm. In one aspect, the tubular base 300-k may have a lip at a distal end 320-g for engaging a distal-most end of a therapeutic or diagnostic device105-f. In various embodiments, the tubular base 300-k, gripping member 1400 and support member 210-j may be monolithically formed.

In some embodiments, the outer surfaces of the gripping members 1400 define an expandable diameter. In a non-expanded state, the expandable diameter may have a diameter that is less than the diameter of the therapeutic or diagnostic device. When the releasable mount device 110-n is mounted on the therapeutic or diagnostic device 105-f, the expandable diameter expands by virtue of the gripping members 1400 compressing towards the tubular base 300-k. In some embodiments, the expanded diameter in an expanded state (i.e., when the gripping members 1400 are compressed to receive the therapeutic or diagnostic device 105-f) is approximately equal to the diameter of the therapeutic or diagnostic device 105-f at a distal end.

In **FIG. 14B**, the releasable mount device 110-n is shown attached to a therapeutic or diagnostic device 105-f in accordance with various embodiments. In various embodiments, a gap 1405 may be formed between the inner surface 330- of the tubular base 300-k and the outer surface of the therapeutic or diagnostic device 105-f. In some embodiments, the gap 1405 corresponds to the compressed height of the gripping member 1400. In various embodiments, the compression force between the therapeutic or diagnostic device 105-f and the gripping member 1400 is substantially uniform. In general, the clamping force increases as the compression force increases or with an increase in compression of the gripping members 1400.

In various embodiments, the tubular base 300-k and the gripping members 1400 are shaped and dimensioned to accommodate or receive therapeutic or diagnostic devices of various sizes. In various embodiments, a system includes a plurality of tubular bases 300-k and gripping members 1400, each shaped and dimensioned based on a therapeutic or diagnostic device size. In practice, a clinician may select one of a plurality of tubular bases 300-k based on the therapeutic or diagnostic device size to be used. In various embodiments, the gap 1405 may be larger in the case of a one size fits all tubular base 300-k.

As shown in FIGs. 14A-14B, the support member 210-j may include a rigid support surface and have an essentially planar shape. In various embodiments, the support member 210-j extends from the proximal end 325-c of the tubular base 300-k to approximately just past the mid-section of the tubular base 300-k and may include a base member 900-c that is positioned underneath the support member 210-j. The base member 900-c illustrated in FIGs. 14A and 14B may be similar to the base members described in FIGs. 9-11 and may extend from the exterior surface of the tubular base 300-k to the bottom surface of the support member 210-j. The base member 900-c of FIGs. 14A and 14B differs from FIGs. 9-11 in that it includes a cylindrical shape. In various embodiments, the support member 210-j may comprise a pattern metal layer overlaying a thermoset polyimide. In various embodiments, an operative element may be coupled with the support member 210-j of the releasable mount device 110-n and may comprise a plurality of radio frequency electrode arrays. The operative element may include a recess that corresponds to the shape of the support member 210-j so that the operative element may couple with the support member 210-j in a keying fashion.

The gripping member 1400 may be a compressible spring configured to apply a retaining force to the therapeutic or diagnostic device 105-f as illustrated in FIGs. 14A and 14B. The spring may be formed of a metal, an elastomer, a thermoplastic, a thermoset polymer, a spring steel or shape memory alloy, and a combination of the same. The gripping member 1400 may extend continuously from the distal end 320-g to the proximal end 325-c of the tubular base 300-k and may be essentially parallel to the central longitudinal axis. In various embodiments, the gripping member 1400 may have a curved shape and may include an arcuate leaf spring with a ramped leading edge. The gripping member 1400 may extend radially inward when compressed and extend radially outward when uncompressed. For example, the gripping member 1400 may extend radially inward by more than 0.03 inches. The gripping member 1400 may be a relatively low durometer material or a polyether block amide. In one aspect, the clamping force of the releasable mount device 110-n is sufficient to maintain the releasable mount device in the initial position and location of original attachment during insertion and use of the therapeutic or diagnostic device.

In some embodiments, a gripping member, such as a spring, may be designed such that it provides sufficient force over a diameter range of therapeutic or diagnostic devices. Merely by way of example, one or more springs could engage a 8.6 mm endoscope with one force (e.g., 1 lbf) and engage a 9.8 mm endoscope with a slightly higher force (e.g., 1.2 lbf). This may make for designs that may be compatible with a range of therapeutic or diagnostic devices, though other designs may be compatible over a range of therapeutic or diagnostic device sizes using this technique or others. In some cases, the forces may be low enough to not cause damage and may make it easier to insert or remove the releasable mount device from the therapeutic or diagnostic device, but may be high enough to provide stability during use. Similar designs may be utilized for some embodiments that utilize one or more tubular bases that include a slot. For example, an inner tubular base may make it so that the force exerted by is more equal across a diameter range, though a tubular base with slot in general may be designed to work across a range of therapeutic or diagnostic devices.

In various embodiments, the clamping force relates to moving the releasable mount device 110-n about an x, y and z axis. The clamping force may include a rotational force about an x, y, and z axis. The clamping force may also include an axial force about an x, y, and z axis. In various embodiments, the clamping force is sufficient to limit or reduce movement of the releasable mount device 110-n in at least one direction. In various embodiments, the clamping force is sufficient to limit or reduce movement of the releasable mount device 110-n in two or three directions about the x, y, and z axes. In various embodiments, the clamping force is sufficient to rotate the releasable mount device 110-n a distance of 0.2 inch with a tangential force equal to or greater than 0.8 lb-force (when the therapeutic or diagnostic device is dry) and 0.5 lb-force (when the therapeutic or diagnostic device is lubricated). In various embodiments, the clamping force is sufficient to rotate the releasable mount device 110-n a distance of 2% of the diameter of the therapeutic or diagnostic device. In various embodiments, the axial force required to detach the releasable mount device 110-n from the therapeutic or diagnostic device is greater than or equal to 1.0 lb-force.

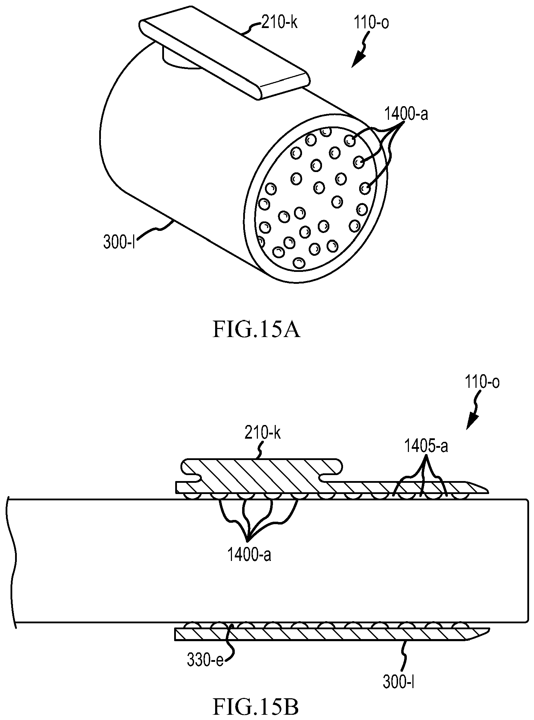

**FIG. 15A** is a perspective view of a releasable mount device 110-o in accordance with various embodiments. The releasable mount device 110-o may have no slot and a gripping member 1440-a including a plurality of beads. **FIG. 15B** is a cross-sectional side view the releasable mount device 110-o shown in FIG. 15A. The releasable mount device 110-o of FIGs. 15A and 15B may include a tubular base 300-1, a support member 210-k, gripping members 1400-a, and a gap 1405-a. The tubular base 300-1, the support member 210-k, and the gap 1405-a may be similar to the releasable mount device 110-n described in FIGs. 14A and 14B. The gripping members 1400-a of the releasable mount device 110-o of FIGs. 15A and 15B differ from the gripping members 1400 of FIGs. 14A and 14B. The releasable mount device 110-o of FIGs. 15A and 15B includes a plurality of gripping members 1400-a attached to the interior surface 330-e of the tubular base 300-1 and extend radially inwardly. In various embodiments, the plurality of gripping members 1400-a may be treads, beads, dots or other types of gripping members.

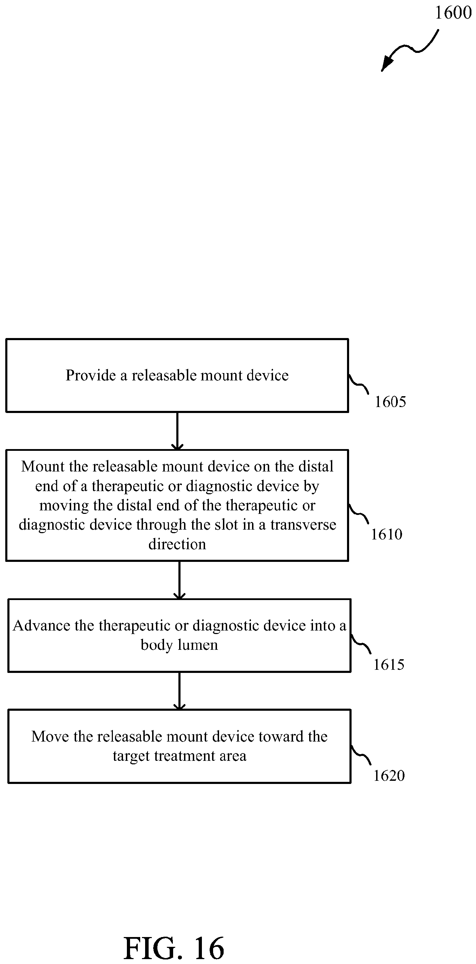

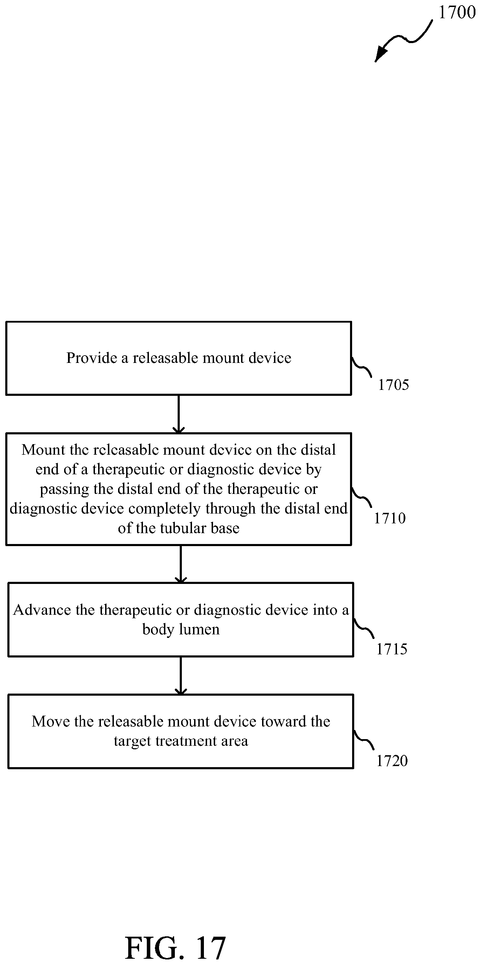

With reference to FIG. 16 and/or FIG. 17, methods of operating a releasable mount device 110 in accordance with the various embodiments will now be described. Referring to **FIG. 16**, a method 1600 may include a step 1605 of providing a releasable mount device, a step 1610 of mounting the releasable mount device on the distal end of a therapeutic or diagnostic device by moving the distal end of the therapeutic or diagnostic device through a slot in the releasable mount device in a transverse direction, a step 1615 of advancing the therapeutic or diagnostic device into a body lumen, and a step 1620 of moving the releasable mount device toward the target treatment area. The releasable mount device may clamp or clip onto the distal end of the therapeutic or diagnostic device when mounted onto the distal end of the therapeutic or diagnostic device.

The releasable mount device of step 1605 may include the releasable mount device having a slot described in greater detail above. The releasable mount device may be selected based on the size of the distal end of the therapeutic or diagnostic device. In step 1610, releasable mount device may be attached to the therapeutic or diagnostic device by moving the therapeutic or diagnostic device through the slot in a transverse direction. The slot of the tubular base may change widths while being mounted to the distal end of the therapeutic or diagnostic device.

After attaching the releasable mount device to the therapeutic or diagnostic device, a step 1615 of advancing the therapeutic or diagnostic device towards a target treatment area, such as by passing the therapeutic or diagnostic device through a body lumen, may be carried out. In step 1620, the releasable mount device may be moved toward the target treatment area. In this manner, an operative element that may coupled with the releasable mount device may be moved toward a target treatment site. Once the operative element is positioned at the target treatment site, the operative element may be operated or activated to perform the intended therapy or diagnosis. Thereafter, the operative element may be withdrawn from the treatment site and removed from the patient. The releasable mount device - still in the original or first position on the therapeutic or diagnostic device - may then be removed from the therapeutic or diagnostic device.

Referring to **FIG. 17**, a method 1700 may include a step 1705 of providing a releasable mount device, a step 1710 of mounting the releasable mount device on the distal end of a therapeutic or diagnostic device by passing the distal end of the therapeutic or diagnostic device completely through the distal end of the tubular base, a step 1715 of advancing the therapeutic or diagnostic device into a body lumen, and a step 1720 of moving the releasable mount device toward the target treatment area.

The releasable mount device of step 1705 may include the releasable mount device having tubular base with no slot described in greater detail above. The releasable mount device may be selected based on the size of the distal end of the therapeutic or diagnostic device. In step 1710, releasable mount device may be attached to the therapeutic or diagnostic device by passing the distal end of the therapeutic or diagnostic device completely through the distal end of the tubular base. In various embodiments, the attaching may include passing the distal end of the therapeutic or diagnostic device completely through and extending past the distal end of the tubular base. The attaching may include inserting the distal end of the therapeutic or diagnostic device through the tubular base in a direction parallel with the longitudinal axis of the tubular base. The tubular base may mount to a distal end of a therapeutic or diagnostic device, and includes a proximal end, a distal end and a central longitudinal axis. A diameter of the tubular base may be larger than the diameter of the distal end of the therapeutic or diagnostic device. The gripping member may be positioned on an inner surface of the tubular base, may include a friction surface, and may extend from the inner surface radially inwardly.

After attaching the releasable mount device to the therapeutic or diagnostic device, a step 1715 of advancing the therapeutic or diagnostic device towards a target treatment area, such as by passing the therapeutic or diagnostic device through a body lumen, may be carried out. In step 1720, the releasable mount device may be moved toward the target treatment area. In this manner, an operative element that may be coupled with the releasable mount device may be moved toward a target treatment site. Once the operative element is positioned at the target treatment site, the operative element may be operated or activated to perform the intended therapy or diagnosis. Thereafter, the operative element may be withdrawn from the treatment site and removed from the patient. The releasable mount device - still in the original or first position on the therapeutic or diagnostic device - may then be removed from the therapeutic or diagnostic device.

The foregoing descriptions of specific embodiments have been presented for purposes of illustration and description. They are not intended to be exhaustive or to limit the various embodiments to the precise forms disclosed, and obviously many modifications and variations are possible in light of the above teaching. The embodiments were chosen and described in order to best explain the principles of the various embodiments and their practical application, to thereby enable others skilled in the art to best utilize the invention and various embodiments with various modifications as are suited to the particular use contemplated. It is intended that the inventive scope to be defined by the Claims appended hereto and their equivalents.

## Claims

1. An apparatus comprising:
a therapeutic or diagnostic device (105) having a distal end (205); and
a releasable mount device (110) mounted on the distal end of the therapeutic or diagnostic device and coupling an operative element (215) with the distal end of the therapeutic or diagnostic device (105), the releasable mount device comprising:
a tubular base (300) coupled with the distal end of the therapeutic or diagnostic device, the tubular base having a proximal end (325), a distal end (320), and a central longitudinal axis, and further comprising a slot (340) extending in a longitudinal direction between the distal end and the proximal end of the tubular base, wherein at least one longitudinal edge (305) of the tubular base defining the slot has a rounded surface;
and
a support member (210) coupled with the tubular base;
the apparatus further comprising an operative element (215) coupled with the support member (210),
**characterized in that**:
the releasable mount device (110) further comprises at least one gripping member (1400) positioned in an interior surface of the tube base (300), the at least one gripping member extending radially inwardly from the interior surface of the tubular base (300), extending longitudinally from the distal to the proximal end of the tubular base and each gripping member comprising a friction surface (350); and
wherein the interior diameter of the tubular base (300) is larger than the diameter of the distal end (205) of the therapeutic or diagnostic device (105);
and
the said at least one longitudinal edge (305) comprises a contact member (335) located along the entire length of the at least one longitudinal edge, wherein the contact member extends away from the interior surface of the tubular base to form a radiused contact surface that contacts an outer surface of the therapeutic or diagnostic device.

2. The apparatus of claim 1, wherein the tubular base (300) is configured to radially expand whereby a slot width of the slot (340) increases from a first width to a second width, the first width being less than a diameter of the distal end (205) of the therapeutic or diagnostic device (105) and the second width being at least equal to the diameter of the distal end of the therapeutic or diagnostic device.

3. The apparatus of claim 1, wherein the support member (210) comprises a foot portion (360) located at the proximal end (325) of the tubular base (300) and a ridge portion (355) extending from the foot portion to the distal end (320) of the tubular base.

4. The apparatus of claim 3, wherein the operative element (215) includes a recess configured to receive the ridge portion (355) of the support member (210).

5. The apparatus of claim 1, wherein the support member (210) is coupled with the tubular base (300) at a location opposite the slot (340).

6. The apparatus of claim 1, wherein the support member (210) pivotably supports the operative element (215).

7. The apparatus of any preceding claim, wherein the friction surface (350) is a member selected from a group consisting of a compressible foam, a low durometer pad, a tacky adhesive, and a combination of the same.

8. The apparatus of any preceding claim, further comprising an exterior coating (700) disposed on the tubular base (300) and the support member (210), wherein the exterior coating comprises at least a gel, a polymer, or a bioactive agent.

9. The apparatus of any preceding claim, wherein the operative element (215) comprises an ablation device.

10. The apparatus of claim 9, wherein the ablation device comprises a plurality of radiofrequency electrode arrays for delivering sufficient energy to a tissue region in an alimentary tract to ablate the tissue region.

11. The apparatus of any preceding claim, wherein at least one longitudinal edge (305) of the tubular base (300) defining the slot (340) is configured to deflect in response to a contact force.

## Patentansprüche

1. Vorrichtung umfassend:
eine therapeutische oder diagnostische Vorrichtung (105) mit einem distalen Ende (205); und
eine lösbare Befestigungsvorrichtung (110), die an dem distalen Ende der therapeutischen oder diagnostischen Vorrichtung befestigt ist und ein operatives Element (215) mit dem distalen Ende der therapeutischen oder diagnostischen Vorrichtung (105) koppelt, wobei die lösbare Befestigungsvorrichtung umfasst:
eine röhrenförmige Basis (300), die mit dem distalen Ende der therapeutischen oder diagnostischen Vorrichtung gekoppelt ist, die röhrenförmige Basis mit einem proximalen Ende (325), einem distalen Ende (320) und einer zentralen Längsachse, und weiter umfassend einen Schlitz (340), der sich in einer Längsrichtung zwischen dem distalen Ende und dem proximalen Ende der röhrenförmigen Basis erstreckt, wobei zumindest eine Längskante (305) der röhrenförmigen Basis, die den Schlitz definiert, eine abgerundete Oberfläche aufweist; und
ein Stützelement (210), das mit der röhrenförmigen Basis gekoppelt ist;
wobei die Vorrichtung weiter ein operatives Element (215) umfasst, das mit dem Stützelement (210) gekoppelt ist,
**dadurch gekennzeichnet, dass**:
die lösbare Befestigungsvorrichtung (110) weiter zumindest ein Greifelement (1400) umfasst, das in einer Innenfläche der röhrenförmigen Basis (300) positioniert ist, wobei sich das zumindest eine Greifelement von der Innenfläche der röhrenförmigen Basis (300) radial nach innen erstreckt, sich in Längsrichtung von dem distalen zu dem proximalen Ende der röhrenförmigen Basis erstreckt und jedes Greifelement eine Reibungsfläche (350) umfasst; und
wobei der Innendurchmesser der röhrenförmigen Basis (300) größer als der Durchmesser des distalen Endes (205) der therapeutischen oder diagnostischen Vorrichtung (105) ist; und
die zumindest eine Längskante (305) ein Kontaktelement (335) umfasst, das sich entlang der gesamten Länge der zumindest einen Längskante befindet, wobei sich das Kontaktelement von der Innenfläche der röhrenförmigen Basis weg erstreckt, um eine abgerundete Kontaktfläche zu bilden, die eine Außenfläche der therapeutischen oder diagnostischen Vorrichtung kontaktiert.

2. Vorrichtung nach Anspruch 1, wobei die röhrenförmige Basis (300) konfiguriert ist, um radial zu expandieren, wobei sich eine Schlitzbreite des Schlitzes (340) von einer ersten Breite zu einer zweiten Breite vergrößert, wobei die erste Breite kleiner als ein Durchmesser des distalen Endes (205) der therapeutischen oder diagnostischen Vorrichtung (105) ist und die zweite Breite zumindest gleich dem Durchmesser des distalen Endes der therapeutischen oder diagnostischen Vorrichtung ist.

3. Vorrichtung nach Anspruch 1, wobei das Stützelement (210) einen Fußabschnitt (360), der sich an dem proximalen Ende (325) der röhrenförmigen Basis (300) befindet, und einen Kammabschnitt (355) umfasst, der sich von dem Fußabschnitt zu dem distalen Ende (320) der röhrenförmigen Basis erstreckt.

4. Vorrichtung nach Anspruch 3, wobei das operative Element (215) eine Aussparung aufweist, die konfiguriert ist, um den Kammabschnitt (355) des Stützelements (210) zu empfangen.

5. Vorrichtung nach Anspruch 1, wobei das Stützelement (210) an einem Ort gegenüber des Schlitzes (340) mit der röhrenförmigen Basis (300) gekoppelt ist.

6. Vorrichtung nach Anspruch 1, wobei das Stützelement (210) das operative Element (215) schwenkbar stützt.

7. Vorrichtung nach einem der vorstehenden Ansprüche, wobei die Reibungsfläche (350) ein Element ist, das aus der Gruppe ausgewählt wird, die aus einem komprimierbaren Schaum, einem Niedrig-Durometer-Pad, einem klebrigen Klebstoff und einer Kombination derselben besteht.

8. Vorrichtung nach einem der vorstehenden Ansprüche, die weiter eine Außenbeschichtung (700) umfasst, die an der röhrenförmigen Basis (300) und dem Stützelement (210) angeordnet ist, wobei die Außenbeschichtung zumindest ein Gel, ein Polymer oder einen bioaktiven Wirkstoff umfasst.

9. Vorrichtung nach einem der vorstehenden Ansprüche, wobei das operative Element (215) eine Ablationsvorrichtung umfasst.

10. Vorrichtung nach Anspruch 9, wobei die Ablationsvorrichtung eine Vielzahl von Hochfrequenz-Elektrodenanordnungen für eine ausreichende Energiezufuhr zu einer Geweberegion in einem Verdauungstrakt, um die Geweberegion abzutragen, umfasst.

11. Vorrichtung nach einem der vorstehenden Ansprüche, wobei die zumindest eine Längskante (305) der röhrenförmigen Basis (300), die den Schlitz (340) definiert, konfiguriert ist, um sich als Reaktion auf eine Kontaktkraft auszulenken.

## Revendications

1. Appareil comprenant :
un dispositif thérapeutique ou diagnostique (105) ayant une extrémité distale (205) ; et
un dispositif de montage amovible (110) monté sur l'extrémité distale du dispositif thérapeutique ou diagnostique et couplant un élément fonctionnel (215) avec l'extrémité distale du dispositif thérapeutique ou diagnostique (105), le dispositif de montage amovible comprenant :
une base tubulaire (300) couplée à l'extrémité distale du dispositif thérapeutique ou diagnostique, la base tubulaire ayant une extrémité proximale (325), une extrémité distale (320), et un axe longitudinal central; et comprenant en outre une fente (340) s'étendant dans une direction longitudinale entre l'extrémité distale et l'extrémité proximale de la base tubulaire, dans lequel au moins un bord longitudinal (305) de base tubulaire définissant la fente a une surface arrondie ; et
un membre de support (210) couplé à la base tubulaire ;
l'appareil comprenant en outre un élément fonctionnel (215) couplé au membre de support (210),
**caractérisé en ce que** :
le dispositif de montage amovible (110) comprend en outre au moins un élément de préhension (1400) positionné dans une surface intérieure de la base tubulaire (300), l'au moins un élément de préhension s'étendant radialement vers l'intérieur à partir de la surface intérieure de la base tubulaire (300), s'étendant longitudinalement depuis l'extrémité distale à proximale de la base tubulaire et chaque élément de préhension comprenant une surface de friction (350) ; et
dans lequel le diamètre intérieur de la base tubulaire (300) est plus grand que le diamètre de l'extrémité distale (205) du dispositif thérapeutique ou diagnostique (105) ; et
et ledit au moins un bord longitudinal (305) comprend un membre de contact (335) situé le long de la longueur entière de l'au moins un bord longitudinal, dans lequel le membre de contact s'éloigne de la surface intérieure de la base tubulaire pour former une surface de contact arrondie qui entre en contact avec une surface extérieure du dispositif thérapeutique ou diagnostique.

2. Appareil selon la revendication 1, dans lequel la base tubulaire (300) est configurée pour s'étendre radialement moyennant quoi une largeur de fente de la fente (340) augmente d'une première largeur à une seconde largeur, la première largeur étant inférieure à un diamètre de l'extrémité distale (205) du dispositif thérapeutique ou diagnostique (105) et la seconde largeur étant au moins égale au diamètre de l'extrémité distale du dispositif thérapeutique ou diagnostique.

3. Appareil selon la revendication 1, dans lequel le membre de support (210) comprend une partie de pied (360) située sur l'extrémité proximale (325) de la base tubulaire (300) et une partie de crête (355) s'étendant de la partie de pied à l'extrémité distale (320) de la base tubulaire.

4. Appareil selon la revendication 3, dans lequel l'élément fonctionnel (215) inclut une cavité configurée pour recevoir la partie de crête (355) sur le membre de support (210).

5. Appareil selon la revendication 1, dans lequel le membre de support (210) est couplé à la base tubulaire (300) sur un emplacement opposé à la fente (340).

6. Appareil selon la revendication 1, dans lequel le membre de support (210) supporte de façon à pouvoir pivoter l'élément fonctionnel (215).

7. Appareil selon une quelconque revendication précédente, dans lequel la surface de friction (350) est un membre sélectionné dans un groupe consistant en une mousse compressible, un tampon de faible dureté, un adhésif et une combinaison de ceux-ci.

8. Appareil selon une quelconque revendication précédente, comprenant en outre un revêtement extérieur (700) disposé sur la base tubulaire (300) et le membre de support (210), dans lequel le revêtement extérieur comprend au moins un gel, un polymère, ou un agent bioactif.

9. Appareil selon une quelconque revendication précédente, dans lequel l'élément fonctionnel (215) comprend un dispositif d'ablation.

10. Appareil selon la revendication 9, dans lequel le dispositif d'ablation comprend une pluralité de réseaux d'électrodes de radiofréquence pour délivrer une énergie suffisante à une région tissulaire dans une voie digestive pour procéder à l'ablation de la région tissulaire.

11. Appareil selon une quelconque revendication précédente, dans lequel au moins un bord longitudinal (305) de la base tubulaire (300) définissant la fente (340) est configuré pour être dévié en réponse à une force de contact.
